# EUROPEAN PATENT APPLICATION

(11) **EP 2 166 357 A1**
(43) Date of publication of application: **24.03.2010**
(21) Application number: 08168828.5
(22) Date of filing: 17.08.2006
(51) Int. Cl.: G01N 33/574

(54) **Protein marker for diagnosing colorectal cancer**

(30) Priority: 18.08.2005 DK 200501162; 11.01.2006 DK 200600047; 18.08.2005 US 709207 P
(62) Divisional of application: 06795263.0
(71) Applicant: ZADEC APS, 5230 Odense M (DK)
(72) Inventor: Larsen, Peter Mose, 5260 Odense S (DK); Fey, Stephen, 5491 Blommeslyst (DK)
(74) Representative: Orsnes, Henrik Egede

(57) **Abstract**

This invention relates to a method for diagnosing colorectal cancer (CRC) in a mammal, such as a human, the method comprising determining an increased level of expression of HSP 90-beta (P08238) in a biological sample from said mammal. It is demonstrated that the combination of metabolic labelling of colon biopsies from both normal and tumour tissues followed by 2 dimensional gel electrophoresis and mass spectrometry is a powerful tool in the identification of marker proteins involved in the development of CRC.

## Description

### FIELD OF THE INVENTION

This invention relates generally to proteins expressed in connection with colorectal cancer, methods of identifying such proteins, methods for screening for drugs, which affect the expression or activity of such proteins, and therapeutic compounds for the treatment and prevention of colorectal cancer.

### BACKGROUND OF THE INVENTION

Colorectal cancer (CRC) is the third leading type of cancer, and the second leading cause of cancer-related death in developed countries, such as the US. It has been estimated that the lifetime risk for developing CRC approaches 6% for men and women. CRC is a suitable disease for screening as it is a common malignancy with a long asymptomatic preclinical phase and a high survival rate if detected in its early stages. Since accumulated evidence strongly suggests that carcinomas arise from so-called adenomas, prevention of CRC should be achievable by screening programs that detect adenomas and lead to their removal.

Colorectal cancer (CRC) is a term used to refer to cancer that develops in the colon or the rectum. The colon and rectum are parts of the digestive system, which is also called the gastrointestinal, or GI, system. The digestive system processes food for energy and rids the body of solid waste matter (fecal matter or stool). After food is chewed and swallowed, it travels through the esophagus to the stomach. There it is partly broken down and then sent to the small intestine, also known as the small bowel. The word "small" refers to the diameter of the small intestine, which is narrower than that of the large bowel. Actually the small intestine is the longest segment of the digestive system. The small intestine continues breaking down the food and absorbs most of the nutrients. The small bowel joins the colon in the right lower abdomen. The colon is divided into three regions: the ascending (right), transverse, and descending (left). The colon tehn joins the rectum in the left lower abdomen. The function of the colon is to continue to absorb water and mineral nutrients from the food matter and serves as a storage place for waste matter. The waste matter left after this process is feces and goes into the rectum, the final part of the digestive system. From there it passes out of the body through the anus.

The wall of the colon and rectum has several layers of tissue. CRC starts in the innermost layer and can grow through some or all of the other layers. Knowing a little about these layers is important, because the stage (extent of spread) of a colorectal cancer depends to a great degree on how deeply it invades into these layers. Colon cancer and rectal cancer, collectively known as colorectal cancer (CRC), have many features in common and will be discussed together in here. In most people, colorectal cancers develop slowly over a period of several years. Before a true cancer develops, a growth of tissue or tumour usually begins as a non-cancerous polyp, which may eventually change into cancer. A polyp develops on the lining of the colon or rectum. Certain kinds of polyps, called adenomatous polyps or adenomas, have the potential to become cancerous. There are other kinds of polyps called hyperplastic and inflammatory polyps. Inflammatory polyps and hyperplastic polyps, in general, are not considered precancerous. But some doctors think that some hyperplastic polyps might be precancerous or a sign that the person may be more likely to develop adenomatous polyps and cancer, particularly if they grow in the right or ascending colon. Another kind of precancerous condition is called dysplasia. This is usually seen in people with diseases, such as ulcerative colitis, which cause chronic inflammation of the colon.

Once cancer forms within a polyp, it can eventually begin to grow into the wall of the colon or rectum. Once they are in the wall, the cancer cells can grow into blood vessels or lymph vessels. Lymph vessels are thin, tiny channels that carry away extracellular fluid and white blood cells that have migrated out of the blood vessels. They first drain into nearby lymph nodes, which are bean-shaped structures that help fight against infections. When the cancer cells spread into blood vessels, they can travel to distant parts of the body. This process of spread is called metastasis. More than 95% of colorectal cancers are adenocarcinomas. These are cancers of the glandular cells that line the inside layer of the wall of the colon and rectum. Other less common types of tumours may also develop in the colon and rectum, such as:
- carcinoid tumours -- these tumours develop from specialized hormone-producing cells of the intestine.
- gastrointestinal stromal tumours -- these tumours develop from specialized cells in the wall of the colon called the "interstitial cells of Cajal." Some are benign (non-cancerous); others are malignant (cancerous). Although these cancers can be found anywhere in the gastrointestinal tract, they are unusual in the colon.
- lymphomas -- these are cancers of immune system cells that typically develop in lymph nodes but also may start in the colon and rectum or other organs.

CRC is usually observed in one of three specific patterns: sporadic, inherited, or familial.

Sporadic disease, with no familial or inherited predisposition, accounts for approximately 70% of colorectal cancer in the population. Sporadic colon cancer or CRC is common in persons older than 50 years of age, probably as a result of dietary and environmental factors as well as normal aging.

Fewer than 10% of patients have an inherited predisposition to colon cancer. The inherited syndromes include those in which colonic polyps are a major manifestation of disease and those in which they are not. The polyposis syndromes are subdivided into familial adenomatous polyposis and the hamartomatous polyposis syndromes. The nonpolyposis predominant syndromes include hereditary nonpolyposis colorectal cancer (HNPCC) (Lynch syndrome I) and the cancer family syndrome (Lynch syndrome II). Although uncommon, these syndromes provide insight into the biology of all types of colorectal cancer.

The third and least understood pattern of CRC development is known as familial colon cancer. In affected families, colon cancer develops too frequently to be considered sporadic colon cancer but not in a pattern consistent with an inherited syndrome. Up to 25% of all cases of colon cancer may fall into this category.

The molecular basis for sporadic colon cancer follows as a multistep model of carcinogenesis. This model describes an accumulation of genetic events, each conferring a selective growth advantage to an affected colon cell. These changes ultimately result in uninhibited cell growth, proliferation, and clonal tumour development. The cumulative effect of these somatic mutations is the cause of sporadic CRC. Four main conclusions are drawn from this model:
1) Colorectal cancer results from the mutational activation of oncogenes and the inactivation of tumour suppressor genes;
2) somatic mutations in at least four or five genes of a cell are required for malignant transformation;
3) the accumulation of multiple genetic mutations rather than the sequence of mutations determines the biological behavior of the tumour, although mutations in the APC gene usually occur early in the process and mutations of the *p53* suppressor gene usually occur late in the process; and
4) features of the tumourigenic process of colon cancer are applicable to other solid tumours, such as breast and pancreatic cancer.

The most commonly inherited colon cancer syndromes are familial adenomatous polyposis and HNPCC. Each of these syndromes is the result of a specific germline mutation. In familial adenomatous polyposis, the germline mutation is always the APC gene, a tumour suppressor gene.

In HNPCC, one of the MMR genes is mutated, most commonly *hMLH1* or *hMSH2*. Several of the hamartomatous polyp syndromes have recently been associated with germline mutations. One example is the Peutz-Jeghers syndrome, which results from an abnormality of the *STK11* tumour suppressor gene.

A number of screening programs for CRC are readily available, including fecal occult blood testing (FOBT), flexible sigmoidoscopy, barium enema, and colonoscopy. Newer methods such as virtual colonoscopy and fecal DNA testing are actively being investigated. The optimal method to detect CRC remains to be developed.

There are potential benefits of barium enema (Double-contrast barium enema; DCBE) when compared to some of the other modalities for screening, such as lower cost (compared to colonoscopy) and less time lost from work. Some point to the improved ability of DCBE to better localize lesions to special anatomic segments of the colon, which can be difficult with colonoscopy. This can be an important issue for those requiring surgery. Finally, DCBE is a safe test, with an estimated perforation rate of one in 25,000, and a mortality rate from perforation of one in 250,000. These potential benefits have to be considered in the context of the downsides of DCBE, which include lack of therapeutic capability, lower sensitivity, and increased patient discomfort when compared with colonoscopy.

Colonoscopy was first discussed as an average risk screening test for CRC in 1988, and has since become a widely endorsed method of screening and even the preferred test of some societies such as the American College of Gastroenterology.

Advances in the understanding of the molecular events that occur during the transformation of adenomas to CRC have led to the development of assays that detect mutations in DNA shed in stool. The DNA in stool arises from bacteria and the normal exfoliation from colonic mucosa, as well as from polyps and CRC. Small amounts of tumour DNA in stools can be amplified using molecular biologic techniques. In recent years, several groups of investigators have successfully isolated DNA in stool from patients with CRC. Based on pooled results from available studies, fecal DNA testing has an overall sensitivity for invasive CRC of 64%, specificity of 95%, and sensitivity for advanced adenomas of 20%. Currently there are no published data on fecal DNA testing in the general population. Fecal DNA testing is appealing because it is neoplasm specific, non-invasive, requires no bowel preparation or dietary restrictions, and has the potential capability of detecting neoplasia throughout the entire length of colon using a single stool collection. The current limitations include the lack of data from screening populations, the need for test refinement to determine how many or which markers are necessary, and expense of testing.

As mentioned, the current cost of fecal DNA testing is high and far exceeds that of FOBT. Limiting the number of tumour markers tested can reduce costs, but possibly at the expense of reduced sensitivity.

Recently the American Cancer Society's (ACS) Colorectal Cancer Advisory Group has reviewed several of the screening methods available (e.g. colonoscopy, fecal occult blood tests (FOBT), immunochemical stool testing, capsule video endoscopy and others) and concluded that the only technology that can be recommended for CRC screening is immunochemical stool testing but there is a great need for improved methodology which can detect the tumours at an early stage (Levin B, et al, CA Cancer J Clin. 2003. 53(1):44-55).

Recognising the need for improved diagnosis which could be used for large scale frequent (annual or biannual) screening, several groups have been working on identifying diagnostic markers either in tissue samples or in the blood.

For example, recently prothymosin-alpha has been identified as a potential biomarker for colon cancer biopsy cells using novel chromatography techniques coupled with SELDI mass spectrometry. Another potential marker for prognosis is thioredoxin-1 since its level of expression in tissue samples correlates well with the stage of colon cancer. Serum thymidine phosphorylase has been suggested as a novel marker to predict the occurrence of hematogenous metastasis in patients with resectable CRC. Monoclonal antibodies have also been used to detect the plasma levels of CD105 protein (a modulator of angiogenesis) and its ligand TGF β and propose a prognosis for patients with CRC. However, a common characteristic of the tests currently used is their relatively low predictive value, so that they need to be complemented with other procedures such as biopsy and/or endoscopy. Considering the drawbacks of the existing screening methods, it can be concluded that multiple protein markers will be needed in order to diagnose with a very high degree of confidence.

The most common ways of treating CRC are by surgical removal of the tumour, or by chemo- and radio-therapy. Fluorouracil (5-FU) with leucovorin (LV) are commonly used in chemotherapy and recently a number of new agents have been introduced including irinotecan (CPT-11) and oxaliplatin which have resulted in improvements to the therapy. A new era is beginning where treatment moves away from chemotherapeutics and replaces them with novel specific targeting agents. Recently completed or ongoing clinical trials using these agents (like anti-angiogenesis drugs, tyrosine kinase inhibitors, and epidermal growth factor blockers) have provided encouraging preliminary data.

As appears from the below cited prior art it is known that proteome analysis enables the identification of various proteins and their association to CRC. These proteins, in themselves, either up-regulated or down-regulated, are indicators of CRC in a patient. The pattern of regulation of a grouping of these proteins also serves as an indicator of CRC. These proteins can be used as targets for the treatment of CRC or for treatment itself.

WO 05015224 discloses a method for the diagnosis of colorectal cancer comprising the steps of a) providing a liquid sample obtained from an individual, b) contacting said sample with a specific binding agent for 60S acidic ribosomal protein PO (RLA-O) under conditions appropriate for formation of a complex between said binding agent and RLA-O, and c) correlating the amount of complex formed in (b) to the diagnosis of colorectal cancer.

WO 04090550 discloses a method of diagnosing colorectal cancer in human samples using several protein markers, including 60S acidic ribosomal protein PO (RLA-O). Differential expression pattern of these markers are indicative of a person having colorectal cancer and/or predictive of the stage of the disease in a colorectal cancer patient. The markers have been identified by assaying a number of tissue and serum samples from healthy individuals and persons diagnosed with colorectal cancer by means of protein chip technology using mass spectrometry.

WO 04079368 discloses specific proteomic approaches to identify proteins, including HSP90, that are expressed in cancer cells. Each marker has been identified by up-regulation of expression in colorectal tumour samples. Using a process whereby proteins were recovered from frozen sections of donor tissues selected from a bank of fresh frozen tissues on the basis of optimal tumour histology and cellularity, it was possible to derive protein profiles of selected sets of normal colon tissue and advanced colorectal carcinomas selected from patients.

WO 00055633 discloses the identification of certain expression profiles and the nucleic acids involved in colorectal cancer, and to the use of such expression profiles and nucleic acids in diagnosis and prognosis of colorectal cancer. The identification has been achieved through proteomics techniques, such as mass spectroscopy assays, 2D gel electrophoresis assays, etc The reference further discloses methods for identifying and using candidate agents and/or targets which modulate CRC.

WO 02078636 discloses methods and compositions for the detection and/or treatment of CRC. More specifically, the reference discloses colorectal cancer- associated proteins and nucleic acids encoding such proteins, which represent cellular markers for colorectal cancer detection and molecular targets for colorectal cancer therapy.

WO 04021010 discloses methods of diagnosing colon and gastric cancers by measuring various expression levels in tissues from patients suffering from these diseases. The expression level is determined by methods such as: (a) detecting the mRNA of the colon cancer-associated genes, (b) detecting the protein encoded by the colon cancer-associated genes, and (c) detecting the biological activity of the protein encoded by the colon cancer-associated genes.

WO 04071267 discloses the use of nicotinamide N-methyltransferase (NNMT) in the diagnosis of colorectal cancer. Furthermore, it discloses a method for diagnosis of colorectal cancer from a stool sample, derived from an individual by measuring NNMT in that sample. Measurement of NNMT can, e.g., be used in the early detection or diagnosis of colorectal cancer.

WO 05015234 discloses use of the protein SAHH (S-adenosylhomocysteine hydrolase) in the diagnosis of colorectal cancer. Furthermore, it especially relates to a method for diagnosis of colorectal cancer from a liquid sample, derived from an individual by measuring SAHH in said sample. Measurement of SAHH can, e.g., be used in the early detection or diagnosis of colorectal cancer.

Previously, proteome studies have proven to be of limited success in identifying such markers. W09842736 and W09843091 disclose certain differentially-expressed protein markers identified by proteomic analysis of clinical samples following laborious processing intended to enrich tumour epithelial cells by removing stromal cells and connective tissue contaminants. However, more recently, a proteomic comparison of murine normal and neoplastic colon tissues identified no statistically significant differences in protein expression patterns (Core et al, Electrophoresis 21: 1772-81, 2000).

The following studies have also been published relating to some markers found in various cancer tissues: Kuniyasu H. Chihara Y. Kondo H. Ohmori H. Ukai R (2003) Amphoterin induction in prostatic stromal cells by androgen deprivation is associated with metastatic prostate cancer, Oncol Rep. 10(6): 1863-8; Cappello F. Bellafiore M, Palma A, David S. Marciano V, Bartolotta T. Sciume C, Modica G. Farina F. Zummo G. Bucchieri F. (2003) 60KDa chaperonin (HSP60) is over-expressed during colorectal carcinogenesis, European Journal of Histochemistry 47(2): 105-10; Yamamoto S. Tomita Y. Hoshida Y. Sakon M, Kameyama M, Imaoka S. Sekimoto M, Nakamori S. Monden M, Aozasa K. (2004) Expression of valosin-containing protein in colorectal carcinomas as a predictor for disease recurrence and prognosis. Clinical Cancer Research 10: 651-7.

As appears from the above discussion of the prior art there are various possibilities to find diagnostic tumour markers. One of the most straight forward methods is to use the diseased tissue itself but as demonstrated with the present invention the prior art technologies fail to identify important CRC marker proteins as discussed below.

It is therefore a major object of the present invention to identify CRC associated marker proteins, which have not been disclosed in the prior art.

Accordingly, the present invention describes the use of the target proteins listed in Table 1 as markers of CRC, and provides methods for their use in such applications as well as for the treatment of CRC.

### SUMMARY OF THE INVENTION

The present inventors have demonstrated that the combination of metabolic labelling of colon biopsies from both normal and tumour tissues followed by 2 dimensional gel electrophoresis and mass spectrometry is a powerful tool in the identification of proteins involved in the development of CRC.

The present inventors have identified proteins associated with CRC by detecting the presence of the proteins of Table 1 in a biological sample. Typically, the biological sample is selected from the group consisting of urine, feces, blood, CSF, saliva, lymphatic fluids, and tissue. Suitably, the tissue is the epithelial tissue of the colon and/or the rectum. Obviously from a diagnositic viewpoint, urine, feces, saliva or blood are to be preferred.

Proteome analysis applied to such biological materials reveals insight into mechanisms responsible for the development of CRC at the protein level as well as identifying proteins of relevance in the diagnosis or treatment of CRC.

In contrast to the above cited prior art the present inventors have made use of fresh tissue sample, i.e. samples which have not been frozen or otherwise stored that would lead to degradation of some unique protein markers. As soon as the tissue samples are excised, they are transferred immediately into cell culture medium and metabolically labelled with a radioisotope (e.g. a mixture of 16 [¹⁴C]-amino acids or [³⁵S]-methionine), which ensures that any protein that is expressed during the labelling interval, even if present in minute amounts, can be detected by state of the art proteome analysis.

As discussed in detail below, the target proteins of the present invention are of particular use inter alla as diagnostic and prognostic markers of cancers, and in particular CRC.

As with known markers, they may be used for example to assist diagnosing the presence of cancer at an early stage in the progression of the disease and predicting the likelihood of clinically successful outcome, particularly with regard to the sensitivity or resistance of a particular patient's tumour to a chemotherapeutic agent or combinations of chemotherapeutic agents. Furthermore these targets can be used for therapeutic intervention in colorectal and other cancers e.g. to specifically target neoplastic cells and reduce toxicity in healthy tissues, and to provide methods for the evaluation of the ability of candidate therapeutic compounds to modulate the biological activity of cancerous cells from the colon, rectum and other tissues.

Thus the present invention relates to the diagnosis and treatment of cancer, and specifically to the discrimination of neoplastic cells from normal cells on the basis of over-expression of specific tumour antigens and the targeting of treatment through exploitation of the differential expression of these antigens within neoplastic cells.

The invention specifically relates to the detection of one or more proteins ("marker proteins" or "target proteins") that are over-expressed in tumour cells compared with the expression in normal cells (see Table 1).

The invention further provides methods for targeting of therapeutic treatments for cancers by directing treatment against these over-expressed proteins. Methods for achieving this targeting may include, but are not limited to; (i) conjugation of therapeutic drugs to a moieties of target proteins or aptamers that specifically recognises the molecular structure of the target proteins, (ii) exposure of the host immune system to the target proteins or fragments thereof by immunization using proteins, polypeptides, expression vectors or DNA vaccine constructs in order to direct the host immune system against tumour cells in which the target proteins are over-expressed, (iii) modification of the biological activity of the target proteins by small molecule ligands, (iv) exploitation of the biological activity of the target proteins to activate prodrugs, (v) modulation of the expression of the target proteins in cells by methods such as antisense gene silencing, use of small interfering RNA molecules, or the targeting of regulatory elements in the genes encoding the target proteins or regulatory proteins that bind to these elements, (vi) specific modulation of the physical interaction of the target proteins with other components of the cell, with for example a small molecule ligand or an immunoglobulin, in order to exert a therapeutic benefit. The present invention thereby provides a wide range of novel methods for the diagnosis, prognosis and treatment of cancers, including CRC, on the basis of the differential expression of the target proteins. These and other numerous additional aspects and advantages of the invention will become apparent to the skilled artisan upon consideration of the following detailed description of the invention.

As recited in the claims the present invention is based on determining the presence, absence or level of expression of at least one marker protein in a biological sample from said mammal, wherein the marker protein is selected from the group consisting of:
- a protein which is defined in Table 1; and
- a protein which is a modification or a derivative of a protein of Table 1 so as to have at least 80% sequence homology with a protein of Table 1. Said modifications can include, but is not limited to: N' or C' truncations, splicing variants, acylation, acetylation, adenylation, ADP-ribosylationiodination, biotinylation, carbamylation, carboxymethylation, deamidation, esterification, farnesylation, formylation, glutathionation, glycosylation, hydroxylation, liposylation, methylation, myristoleylation, oxidation, palmitoylation, phosphorylation, prenylation, sialation, stearoylation, sulphonation, sulphatation, vitamin C- and K-dependent modifications, and selenoproteins.

However, since the prior art discloses the use of HSP90 and 60S acidic ribosomal protein for the detection of CRC the subject-matter of the present invention is based on the determinination of the presence of at least one further marker protein selected from the group, which is not HSP90 or 60S acidic ribosomal protein, when said at least one marker protein is HSP90 and/or 60S acidic ribosomal protein. This does also apply to the proteins from Group A of Table 1, which are known to be up/down regulated in CRC tissue.

### DETAILED DESCRIPTION OF THE INVENTION

The present invention relates in a first aspect to the proteins identified by 2D-gel electrophoresis and mass spectrometry.

A further aspect relates to the use of proteins of Table 1 as markers or indicators for CRC as well as to the use of proteins whose presence, absence or prevalence has previously not been associated with CRC. The changes in protein expression and patterns of protein expression are considered to be important markers for diagnosis, prognosis and therapeutic applications and targets.

The proteins of the invention may a) be involved in energy transduction and redox potentials and glycolytic enzymes; b) be involved in purine/pyrimidine synthesis, DNA/RNA synthesis, RNA processing, amino acid metabolism and protein synthesis; c) as chaperones; d) be involved in differentiation, apoptosis, regulation and signal transduction; e) be involved in cellular defence; f) be involved in cellular structure; g) be involved in hormone/neurotransmitter metabolism; h) or have other functions.

A first aspect of the invention relates to marker proteins. The marker proteins are selected from the group consisting of: a) a protein of Table 1; and b) a protein with at least 80% sequence homology with a protein in a).

Also, the marker protein can be selected from the group consisting of: a) one or more proteins present in a significantly lower or significantly higher amount on a polyacrylamide gel of proteins from said biological sample in relation to a control; b) one or more proteins present on a polyacrylamide gel of proteins from said biological sample and absent on polyacrylamide gel of proteins of a control; and c) one or more proteins absent on a polyacrylamide gel of proteins from said biological sample and present on polyacrylamide gel of proteins of a control.

The meaning of the term "control" is evident for a person skilled in the art of proteomics. Preferably, the control is an IOD% value of a spot (having the same position) from a gel of proteins from a sample originating from a human who is not predisposed for or having CRC or from a healthy region of colon from a patient with CRC. The term "marker protein" is meant to encompass the above mentioned proteins, as well as similar proteins from other species of mammals that are used for experimentation into CRC. The human forms of the proteins are of particular interest. For example, it is suggested that the rat forms of the human proteins can be used as markers for CRC in rat experimental models. Thus, the term "marker protein" encompasses the mammalian forms of the identified human proteins in Table 1.

Another aspect of the invention relates to a method for diagnosing or for determining the predisposition of CRC in a mammal, e.g. in a human, the method comprising determining the presence, absence or level of expression of at least one marker protein in a biological sample from said mammal. The biological sample can be selected from the group consisting of urine, feces, blood, saliva, lymphatic fluids, and tissue, presently blood, feces, urine, saliva or colon epithelial tissue is preferred.

A presently preferred method comprises establishing the increased expression of at least one marker protein (an up-regulated marker protein) selected from the group consisting of: a) a protein which is defined in Table 1, and marked as up-regulated; and b) a protein which is a modification or a derivative of a protein in a), so as to have at least 80% sequence homology with a protein in a), but of course the invention also relates to a method establishing the decreased expression of at least one down-regulated marker protein.

In another embodiment, the invention relates to a method for determining the predisposition for CRC in a human, the method comprising: a) determining the increased expression of at least one marker protein in a biological sample originating from the human, said marker protein being selected from the group consisting of: i) an up- regulated protein defined in Table 1, or ii) a protein which is a modification or a derivative of a protein in i), so as to have at least 80% sequence homology with a protein in i); b) determining the decreased expression in a biological sample from the human of at least one marker protein, said marker protein being selected from the group consisting of: i) a down-regulated protein defined in Table 1 or ii) a protein which is a modification or a derivative of a protein in i), so as to have at least 80% sequence homology with a protein in i); or c) combinations of the determinations in a) and b).

Thus, the determination of whether a protein is up-regulated or down-regulated serves as useful indicators of CRC susceptibility. The pattern of up- and down-regulation may also serve as an indicator. That is to say that the level of expression of more than one protein is established and the pattern of expression of a grouping of proteins is used as an indicator.

In a suitable embodiment, at least one marker protein is selected from the group consisting of one or more proteins present in a significantly lower or significantly higher amount on a polyacrylamide gel of proteins from said biological sample in relation to a control, one or more proteins present on a polyacrylamide gel of proteins from said biological sample and absent on a polyacrylamide gel of proteins of a control, one or more proteins absent on a polyacrylamide gel of proteins from said biological sample and present on polyacrylamide gel of proteins of a control.

In another embodiment, the invention relates to a method of treating CRC, or preventing or delaying the onset or of CRC, in a mammal, e.g. in a human, comprising altering the expression of a least one marker protein.

The method preferably comprises administering a compound which can alter the expression back towards the control of one or more proteins selected from the group consisting of: a) a protein which is defined in Table 1; b) a protein which is a modification or a derivative of a protein of Table 1, so as to have at least 80% sequence homology with a protein of Table 1; c) a protein having the same function as a protein in a) and/or b); d) a nucleotide sequence coding for a protein in a), b) or c); e) an antibody for a protein of a), b) or c); f) a nucleic acid fragment capable of binding to a protein of a) b) or c); and g) a compound capable of binding to a protein of a), b) or c) to said human.

Also, the invention relates to the use of such a compound for the manufacture of a medication for the treatment or prophylaxis of CRC. The term "colorectal cancer" or its abbreviation "CRC" comprises diseases associated with colorectal cancer.

With regard to a method of treating CRC, a single protein may be targeted for therapy or a group of proteins may be targeted. The level of expression of these targeted proteins may be altered or the proteins themselves may be interfered with in order to alter their activity. Thus, an interesting embodiment of a method of treating CRC in a human comprises altering the expression of a marker protein.

In another embodiment, the invention relates to a method of determining the likelihood of an agent having a therapeutic effect in the treatment of CRC comprising determining the relative level of expression of one or more marker proteins before and after exposing a test model to said agent.

Also, the invention relates to a method of determining the effect of a compound in the treatment of CRC comprising determining the level of expression of proteins of one or more marker proteins.

In a further embodiment, the invention relates to a method of determining the level of effect of a compound used in the treatment of CRC comprising determining the level of expression of one or more marker proteins before and after exposing a test model to said agent.

Also, the invention relates to a method of determining the nature or cause of CRC in a mammal, e.g. in a human, having or susceptible to the disease comprising establishing the level of expression of a at least one marker protein in relation to a model.

Another embodiment of the invention relates to a nucleic acid fragment comprising a nucleotide sequence which codes for a marker protein, or to a nucleic acid fragment which hybridizes with a such a nucleic acid fragment or a part thereof or with its complementary strand. The invention also relates to the use of the above mentioned nucleic acid fragments for detecting the presence of a marker protein.

In yet another embodiment, the invention relates to an antibody able to bind to a marker protein. Such an antibody can be a polyclonal antibody or a monoclonal antibody. The invention also relates to the use of such an antibody for detecting the presence of a marker protein.

In a further embodiment, the invention relates to a test kit for diagnosing CRC or a genetic predisposition for CRC in a mammal, e.g. in a human, comprising: a) a binding means which specifically binds to at least one marker protein; or which binding means is an antibody for at least one said marker protein, a nucleic acid fragment capable of binding to at least one said marker protein, or a compound capable of binding to at least one said marker protein; b) a means for detecting binding, if any, or the level of binding, of the binding means to at least one of the marker proteins or to at least one of the nucleic acid fragments, and c) a means for correlating whether binding, if any, or the level of binding, to said binding means is indicative of the individual mammal having a significantly higher likelihood of having CRC or a genetic predisposition for having CRC.

In another embodiment, the invention relates to a method for determining the effect of a substance, the method comprising using a mammal, e.g. a human, which has been established to be an individual having a high likelihood of having CRC or a genetic predisposition for having CRC by use of a method of the invention, the method comprising administering the substance to the individual and determining the effect of the substance.

The present inventors anticipate that a method of determining the nature or cause of CRC in a human having or susceptible to the disease comprising establishing the level of expression of a protein of Table 1 in relation to a model serves for understanding the disease and potential therapies.

In the testing of compounds, knowledge about the activity or target of an agent is useful for understanding the therapeutic activity of said agent and may assist in improving the desired therapy.

The developments of the present inventors allow for a method of determining the effect of a compound in the treatment of CRC comprising determining the level of expression of one or more marker proteins and to a method of determining the level of effect or level of activity of a compound used in the treatment of CRC comprising determining the level of expression of one or more marker proteins before and after exposing a test model to said agent.

A very important embodiment of the invention relates to a pharmaceutical composition which comprises: a) a substance which is capable of regulating the expression of a nucleic acid fragment coding for at least part of a marker protein; b) a marker protein; c) a derivative, homologue or mimic of a marker protein; d) an antibody for a marker protein; e) a nucleic acid fragment capable of binding to a marker protein; and/or f) a compound capable of binding to a marker protein.

The pharmaceutical composition can be used as a medicament, such as for treatment or prophylaxis of CRC.

It should be noted that the detection of any combination of more than one of the markers would be expected to make the analysis an even more reliable indicator for the disease related to CRC. Thus, a method for diagnosing or determining the predisposition to CRC comprising determining the presence, activity, concentration and/or level of expression of a combination of two markers would be preferred and three or more markers (e.g. at least 4, 5, 6 or 7 markers) would be strongly preferred. Detection of more than one marker protein would be expected to increase both the specificity and the sensitivity of diagnosis and thus the value of the diagnostic test.

It is analogously suggested that treatment with more than one compound (e.g. at least 2, 3, 4, 5, 6 or 7 compounds) according to the invention (e.g. more than one compound chosen from the group consisting of: a polypeptide, a nucleic acid fragment or an antibody according to the invention), said compounds combined being able to affect the level of more than one marker protein, would make the treatment of the disease even more efficient. In this regard, it is possible and even expected that treatment with only one compound will affect the expression of more than one marker protein.

The term "polypeptide" in the present invention should have its usual meaning. That is an amino acid chain of any length, including a full-length protein, oligopeptides, short peptides and fragments thereof, wherein the amino acid residues are linked by covalent peptide bonds. The terms "polypeptide", "peptide", "amino acid sequence" and "protein" are used interchangeably.

The protein may be chemically or biochemically modified by being phosphorylated, methylated, sulphylated, glycosylated or by the addition of any form of lipid or fatty acid, ubiquitin or any other side groups or by containing additional amino acids or any other forms of modification (of which there are over 200 known). These modifications occur at specific sites on the protein and a particular modification at one site can have different effects as the same modification at a different site on the same protein. They can be reversible in the cell where they are used for example to turn on and off enzymes and so the proteins can exist in a variety of forms - each with an associated activity level for each of the proteins functions. Furthermore the polypeptide may be cleaved e.g. by processing at its N- or C-termini to remove signal peptides or the mRNA can be be spliced to remove an internal sequence which when translated will result in an altered protein. Examples of many of these can be found in protein databases like EXPASY and there exists an ever growing range of tools to predict these modifications and their function (see http://www.expasy.ch). Since it is estimated that each protein in man is modified on average 10 - 20 times, it is expected that the majority of the proteins identified here are modified in some way or another.

Each polypeptide may thus be characterized by specific amino acids and be encoded by specific nucleic acid sequences. It will be understood that such sequences include analogues and variants produced by recombinant or synthetic methods wherein such polypeptide sequences have been modified by substitution, insertion, addition or deletion of one or more amino acid residues in the recombinant polypeptide and still be immunogenic in any of the biological assays described herein.

Substitutions are preferably "conservative". Conservative substitutions are known to a person skilled in the art. Preferably, amino acids belonging to the same grouping (nonpolar (G. A, P, I, L and V), polar- uncharged (C, S, T, M, N and Q), polar-charged (D, E, K and R) and aromatic (H, F, W and M) are considered. Within these groups, amino acids may be substituted for each other, but other substitutions are of course possible.

Each polypeptide is encoded by a specific nucleic acid sequence. It will be understood that such sequences include analogues and variants hereof wherein such nucleic acid sequences have been modified by substitution, insertion, addition or deletion of one or more nucleic acid residues (including the insertion of one or more introns (small or large)) . Substitutions are preferably silent substitutions in the codon usage, which will not lead to any change in the amino acid sequence, but may be introduced to enhance the expression of the protein.

In the present context the term "substantially pure polypeptide" means a polypeptide preparation which contains at most 25% by weight of other polypeptide material with which it is natively associated (lower percentages of other polypeptide material are preferred, e.g. at most 20%, at most 15%, at most 10%, at most 5%, and at most 1%). It is preferred that the substantially pure polypeptide is at least 96% pure, i. e. that the polypeptide constitutes at least 96% by weight of total polypeptide material present in the preparation, and higher percentages are preferred, such as at least 97%, at least 98%, at least 99%, at least 99.25%, at least 99.5%, and at least 99.75%. It is especially preferred that the polypeptide fragment is in "essentially pure forms", i.e. that the polypeptide fragment is essentially free of any other protein with which it is natively associated, i.e. free of any other protein from a mammal. This can be accomplished by preparing the polypeptides of the invention by means of recombinant methods in a host cell as known to a person skilled in the art, or by synthesizing the polypeptide fragment by the well-known methods of solid or liquid phase peptide synthesis, e.g. by the method described by Merrifield (Merrifield, R. B. Fed. Proc. Am. Soc. Ex. Biol. 21: 412, 1962 and J. Am. Chem. Soc. 85: 2149/ 1963) or variations thereof, or by means of recovery from electrophoretic gels.

The term "protein" also encompasses derivatives, analogues and mimetics of the above mentioned polypeptides. Such a derivative, analogue and mimetic preferably have the same activity, e.g. the same kind of enzymatic activity, as the polypeptide which it is derived from. The derivative, analogue or mimetic can have a lower level activity, the same level or preferably, a higher level of activity than the parent polypeptide.

The term "a least one" (e.g. at least one compound or at least one marker protein) encompasses the integers 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, and 66. It should be understood that a single marker protein can be used, but it can be advantageous to use more than one marker protein in the methods of the invention, that is to say that the level of expression of more than one protein is established and the pattern of expression of a grouping of proteins is used as an indicator. Obviously the reliability of identification increases as the number in the group increase.

A "peptidomimetic" is a molecule that mimics the biological activity of a peptide but is no longer peptidic in chemical nature. By strict definition, a peptidomimetic is a molecule that no longer contains any peptide bonds (that is, amide bonds between amino acids). However, the term peptidomimetic is sometimes used to describe molecules that are no longer completely peptidic in nature, such as pseudo-peptides, semi-peptides and peptoids. Whether completely or partially non-peptide, peptidomimetics according to this invention provide a spatial arrangement of reactive chemical moieties that closely resembles the three-dimensional arrangement of active groups in the peptide on which the peptidomimetic is based. As a result of this similar active-site geometry, the peptidomimetic has effects on biological systems, which are similar to the biological activity of the peptide. The present invention encompasses peptidomimetic compositions which are analogs that mimic the activity of biologically active peptides according to the invention, i.e. the peptidomimetics can be used for treatment of CRC-related diseases. The peptidomimetic of this invention are preferably substantially similar in both three-dimensional shape and biological activity to the peptides or active sites of such as set forth above.

Alternatively, the mimetic can be an 'antimimetic'. In other words, a molecule that can fit into and block the active site of the protein, or bind to binding sites or sites of interaction with other biological molecules and so interfere with the function of the protein. Most current drugs are of this type. Such antimimetics that are capable of interacting with the polypeptides of the invention are encompassed by the present invention.

There are clear advantages for using a mimetic of a given peptide rather than the peptide itself, because peptides commonly exhibit two undesirable properties: (1) poor bioavailability; and (2) short duration of action. Peptidomimetics offer an obvious route around these two major obstacles, since the molecules concerned are small enough to be both orally active and have a long duration of action. There are also considerable cost savings and improved patient compliance associated with peptidomimetics, since they can be administered orally compared with parenteral or transmucosal administration for peptides. Furthermore, peptidomimetics are much cheaper to produce than peptides. Finally, there are problems associated with stability, storage and immunoreactivity for peptides that are not experienced with peptidomimetics.

Thus peptides described above have utility in the development of such small chemical compounds with similar biological activities and therefore with similar therapeutic utilities. The techniques of developing peptidomimetics are conventional. Thus, peptide bonds can be replaced by non-peptide bonds that allow the peptidomimetic to adopt a similar structure, and therefore biological activity, to the original peptide. Further modifications can also be made by replacing chemical groups of the amino acids with other chemical groups of similar structure. The development of peptidomimetics can be aided by determining the tertiary structure of the original peptide by spectroscopy, crystallography and/or computer-aided molecular modelling. These techniques aid in the development of novel compositions of higher potency and/or greater bioavailability and/or greater stability than the original peptide (Dean (1994), BioEssays, 16: 683-687; Cohen and Shatzmiller (1993), J. Mol. Graph. 11: 166-173; Wiley and Rich (1993), Med. Res. Rev., 13: 327-384; Moore (1994), Trends Pharmacol. Sci., 15: 124-129; Hruby (1993), Biopolymers, 33: 1073-1082; Bugg et al. (1993), Sci. Am., 269: 92- 98, all incorporated herein by reference). Once a potential peptidomimetic compound is identified, it may be synthesized and assayed using the diagnostic assay described herein or an appropriate disease suppressor assay (see, Finlay et al. (1983), Cell, 57: 1083-1093 and Fujiwara et al. (1993), Cancer Res., 53: 4129- 4133, both incorporated herein by reference), to assess its activity.

Thus, through use of the methods described above, the present invention provides compounds exhibiting enhanced therapeutic activity in comparison to the polypeptides described above. The peptidomimetic compounds obtainable by the above methods, having the biological activity of the above named peptides and similar three dimensional structure, are encompassed by this invention. It will be readily apparent to one skilled in the art that a peptidomimetic can be generated from any of the modified peptides described previously or from a peptide bearing more than one of the modifications described previously. It will furthermore be apparent that the peptidomimetics of this invention can be further used for the development of even more potent non-peptidic compounds, in addition to their utility as therapeutic compounds.

By the terms "nucleic acid fragment" and "nucleic acid sequence" and the like are understood any nucleic acid molecule including DNA, RNA, LNA (locked nucleic acids), PNA, RNA, dsRNA and RNA-DNA-hybrids. Also included are nucleic acid molecules comprising non-naturally occurring nudeosides. The term includes nucleic acid molecules of any length, e. g. from 10 to 10000 nucleotides, depending on the use. When the nucleic acid molecule is for use as a pharmaceutical, e.g. in DNA therapy, or for use in a method for producing a polypeptide according to the invention, a molecule encoding at least a part of the polypeptide is preferably used, having a length from about 10 to about 1000 nucleotides, the molecule being optionally inserted into a vector. When the nucleic acid molecule is used as a probe, as a primer or in antisense therapy, a molecule having a length of 10-100 is preferably used. According to the invention, other molecule lengths can be used, for instance a molecule having at least 12, 15, 21, 24, 27, 30, 33, 36, 39, 42, 50, 60, 70, 80, 90, 100, 200, 300, 400, 500 or 1000 nucleotides (or nucleotide derivatives), or a molecule having at most 10000, 5000, 4000, 3000, 2000, 1000, 700, 500, 400, 300, 200, 100, 50, 40, 30 or 20 nucleotides (or nucleotide derivatives). It should be understood that these numbers can be freely combined to produce ranges.

The term "stringent" when used in conjunction with hybridization conditions is as defined in the art, i.e. the hybridization is performed at a temperature not more than 15-20°C under the melting point (Tm) of the nucleic acid fragment, cf. Sambrook et al., Molecular Cloning; A laboratory manual, Cold Spring Harbor Laboratories, NY, 1989, pages 11.45 11.49. Preferably, the conditions are "highly stringent", i.e. 5-10°C under the melting point (Tm). In the present invention, the hybridization conditions are preferably stringent. Throughout this specification, unless the context requires otherwise, the word "comprise", or variations thereof such as "comprises" or "comprising", will be understood to imply the inclusion of a stated element or integer or group of elements or integers but not the exclusion of any other element or integer or group of elements or integers.

The term "sequence identity" (or "sequence homology") indicates a quantitative measure of the degree of homology between two amino acid sequences of equal length or between two nucleotide sequences of equal length. If the two sequences to be compared are not of equal length, they must be aligned to best possible fit with the insertion of gaps or alternatively truncation at the ends of the protein sequences. The sequence identity can be calculated as (Nref-Ndif)*100/Nref, wherein Ndif is the total number of non-identical residues in the two sequences when aligned, and wherein Nref is the number of residues in one of the sequences. Hence, the DNA sequence AGTCAGTC will have a sequence identity of 75% with the sequence MTCAATC (Ndif=2 and Nref=8). A gap is counted as non-identity of the specific residue(s), i.e. the DNA sequence AGTG C will have a sequence identity of 75% with the DNA sequence AGTCAGTC (Nd f=2 and Nref=8).

Sequence identity can alternatively be calculated by the BLAST program e. g. the BLAST program (Pearson W.R and D.J. Lipman (1988), PNAS USA 85:2444-2448) (www.ncbi.nim.nih.gov/BLAST/) or the BLAST program available at the ExPaSy website (op. sit.). In one aspect of the invention, alignment is performed with the sequence alignment method ClustalW with default parameters as described by Thompson J., et al, Nucleic Acids Res 1994 22:4673-4680, available at http: /www2.ebi.ac.uk/clustalw/. Alternatively, the degree of homology between two nucleic acid sequences is determined by using GAP version 8 from the GCG package with standard penalties for DNA: GAP weight 5.00, length weight 0.300, Matrix described in Gribskov and Burgess, Nucl. Acids Res. 14(16); 6745-6763 (1986), and the degree of homology between two amino acid sequences is determined by using GAP version 8 from the GCG package (Genetics Computer Group, 575 Science Drive, Madison, Wis. 53711, USA) with standard penalties for proteins: GAP weight 3.00, length weight 0.100, Matrix 25 described in Gribskov and Burgess, Nucl. Acids Res. 14(16); 6745-6763 (1986).

A preferred minimum percentage of sequence homology is at least 70%, such as at least 80%, at least 85%, at least 90%, at least 91 %, at least 92%, at least 93%, at least 94%, at least 95%, at least 96%, at least 97%, at least 98%, at least 99%, and at least 99.5%.

The invention also relates to the use of a polypeptide or nucleic acid of the invention for use as therapeutic vaccines as have been described in the literature exemplified by Lowry, D.B. et al 1999, Nature 400: 269-71.

A monoclonal or polyclonal antibody, which is specifically reacting with a polypeptide of the invention in an immuno assay, or a specific binding fragment of said antibody, is also a part of the invention.

The antibodies can be produced by methods known to a person skilled in the art. The polyclonal antibodies can be raised in a mammal, for example, by one or more injections of a polypeptide according to the present invention and, if desired, an adjuvant. The monoclonal antibodies according to the present invention may, for example, be produced by the hybridoma method first described by Kohler and Milstein, Nature, 256:495 (1975), or may be produced by recombinant DNA methods such as described in U.S. Pat. No. 4,816,567. The monoclonal antibodies may also be isolated from phage libraries generated using the techniques described by McCafferty et al, Nature, 348:552-554 (1990), for example. Methods for producing antibodies are described in the literature, e.g. in US 6,136, 958.

In diagnostics, treatment or testing, an antibody, a nucleic acid fragment and/or a polypeptide of the invention can be used either alone, or as a constituent in a composition. Such compositions are known in the art, and comprise compositions in which the antibody, the nucleic add fragment or the polypeptide of the invention is coupled, preferably covalently, to at least one other molecule, e.g. a label (e.g. radioactive or fluorescent) or a carrier molecule.

The present invention is further directed to methods for using the compounds described above to therapeutically and/or prophylactically treat a patient for a CRC-related disease.

The methods of the present invention include the steps of: a) incorporating one or more of the compounds of the present invention in a suitable pharmaceutical carrier; and b) administering either a therapeutically effective dosage or a prophylactically effective dosage of the compound or compounds incorporated in the carrier to a patient.

The term "suitable pharmaceutical carrier" refers to any carrier known in the pharmaceutical arts for administration of compounds to a patient. Any suitable pharmaceutical carrier can be used according to the present invention, so long as compatibility problems do not arise.

Administration of an effective dosage to a patient can be accomplished by parenteral injection, such as intravenously, intramuscularly or intra-arterially. The compounds can also be administered orally or transdermally, or by any other means known to those skilled in the art, e.g. by means of an inhalator or a nasal spray. Oral administration is presently preferred.

As used herein, the term "therapeutically effective amount" refers to that amount of one or more of the compounds of the present invention required to therapeutically treating a patient. Such treatment is appropriate for subjects having a diagnosed CRC-related disease. Similarly, the term "prophylactically effective amount" refers to that amount of one or more of the compounds of the present invention needed to prophylactically treat a patient. Such treatment is appropriate for subjects who, for example, have not yet established any clinical symptoms of a CRC-related disease. It could be advantageous to start a prophylactic treatment as soon it is determined that the subject is in risk for developing a CRC related disease/ e.g. by means of a determination of a predisposition for CRC by having an altered level of markers.

As will be appreciated by a person skilled in the art, the dosage of a compound given, the route of administration and the duration of therapy will be dependent not only on the type of compound and its effectiveness in treating the disease but also upon the individual being treated, taking into consideration such factors as the body weight of the patient, other therapies being employed to treat the patient, and the condition, clinical response and tolerance of the patient. Dosage, administration, and duration of therapy can be de termined by one skilled in the art upon evaluation of these and other relevant factors.

The invention provides human CRC-mediating proteins, that is, proteins identified as involved in or effected during the development of CRC. CRC-mediating proteins are characterized as proteins whose expression is altered during the development of CRC relative to their expression in the absence of the development of CRC. The present disclosure identifies CRC-mediating proteins from a 2-dimensional gel database of human colon/rectum biopsies and epithelial cell proteins. CRC-mediating proteins include protective CRC-mediating proteins and deleterious CRC-mediating proteins.

The invention provides human CRC-mediating proteins identified through the use of 2-dimensional gels to compare control and cancerous epithelial cells to identify which proteins respond, identifying the proteins which play a role in the cell response. Interlink analysis can be used to define functional groups of proteins and their regulation (e.g., by kinase phosphorylation or other post-translational modifications).

The invention provides substantially purified protective CRC-mediating proteins ("protective proteins") characterized as capable of protecting against development of CRC in a subject at risk for the development of the disease or ameliorating or reducing the symptoms of CRC in a subject suffering from CRC. The protective protein of the invention may act directly to protect against CRC, or may act indirectly by inducing or increasing the synthesis of a second protective protein or by reducing or inhibiting the synthesis of a deleterious protein. The invention further includes amino acid sequences having at least 80%, preferably at least 90%, more preferably at least 95% and most preferred at least 98% identity to the fully length amino acid sequence of a human CRC-mediating protein. Percent homology or identity can be determined, for example, by comparing sequence information using the GAP computer program, version 6.0, available from the University of Wisconsin Genetics Computer Group (UWGCG). The GAP program utilizes the alignment method of Needleman and Wunsch (1970) J. Mol. Biol. 48:443, as revised by Smith and Waterman (1981) Adv. Appl. Math. 2:482. Briefly, the GAP program defines similarity as the number of aligned symbols (i.e. nucleotides or amino acids) which are similar, divided by the total number of symbols in the shorter of the two sequences. The preferred default parameters for the GAP program include: (1) a unitary comparison matrix (containing a value of 1 for identities and 0 for non-identities) and the weighted comparison matrix of Gribskov and Burgess (1986) Nucl. Acids Res. 14:6745, as described by Schwartz and Dayhoff, eds. (1979) Atlas Of Protein Sequence And Structure, National Biomedical Research Foundation, pp. 353-358; (2) a penalty of 3.0 for each gap and an additional 0.10 penalty for each symbol in each gap; and (3) no penalty for end gaps.

The invention further includes polynucleotide sequences encoding the CRC-mediating proteins of the invention, including DNA, cDNA, PNA and RNA sequences. It is also understood that all polynucleotides encoding all or a portion of a CRC-mediating protein are also included herein, as long as they encode a polypeptide with the CRC-mediating activity. Such polynucleotides include naturally occurring, synthetic, and intentionally manipulated polynucleotides.

For example, such a polynucleotide may be subjected to site directed mutagenesis. The polynucleotide sequences of the invention also include antisense sequences. Antisense sequences include sequences synthesized with modified oligonucleotides. The polynucleotides of the invention include sequences that are degenerate as a result of the genetic code. There are 20 natural amino acids, most of which are specified by more than one codon. Therefore, all degenerate nucleotide sequences are included in the invention as long as the amino acid sequence of the CRC-mediating polypeptide is encoded by the nucleotide sequence is functionally unchanged.

Deleterious CRC-mediating proteins ("deleterious proteins") are characterized as enhancing the development of or increasing the risk of a subject developing CRC.

Two-dimensional gel electrophoresis (2-DGE) is a particularly effective tool for separating mixtures of proteins (e.g. Andersen et al. (1995) Diabetes 44:400-407; John NE et al., Diabetes. (2000); 49:1819-29. Christensen et al., Autoimmunity. (2000); 32:1-15 and Mose Larsen et al., Diabetes. (2001); 50: 1056-63). Cell protein extracts are put onto a gel, and the individual proteins are separated first by charge and then by size. The result is a characteristic picture of as many as 1,000 to 5,000 spots, each usually constituting a single protein. Resolution is improved by increasing gel size, and by enhancing the sensitivity through the use of radiolabel methods, silver staining, and the reduction in thickness of the gels to 1.5 mm and less. A further significant improvement in resolution can be obtained by running first dimension gels covering narrow pH ranges (e. g. 1.5, 1 pH units or less). As described in the Examples below, single proteins recovered from 2D gels can be identified by mass spectrometry to obtain a trypsin cleavage pattern as well as the precise molecular weight of each peptide. These observed values are then used to search in DNA and protein databases to determine if matches exist to previously identified proteins. Identity can be determined from a known protein or deduced from high homology to a known protein.

When 2D gel electrophoresis is used to separate and identify protein spots which exhibit an altered synthesis during development of CRC, an identified protein spot is excised from the gel and digested with trypsin to produce peptides. The peptides are recovered from the gel and subjected to mass spectroscopy (matrix assisted laser desorption/ionization mass spectrometry - MALDI) and the resulting MS-profiles are analyzed against the computerized MS-profiles of all sequences found in the public sequence databases, as well as against propriety sequence information. If any matches to previously cloned sequences are obtained, information about the corresponding gene and encoded protein is collected. When an identified CRC-mediating protein does not match a previously cloned protein, the protein may be microsequenced to obtain partial amino acid sequence information by methods known to the art. Proteins (when available in sufficient quantities) may also be partially sequenced by for example nano-electrospray tandem mass spectrometry where particular peptides are fragmented in the gas phase and the molecular weights of the fragments used to derive part of the amino acid sequence. Once partial sequence information is available then it is also possible to search in the cDNA or EST (expressed sequence tag) databases in addition to those mentioned previously.

Based upon results obtained from database searches or amino acid sequencing, specific or degenerate primers are constructed and used to screen human colon and/or rectum epithelial libraries or first-strand cDNA by PCR is used to clone partial sequences of the corresponding cDNA.

The obtained sequences are then used to obtain full-length coding regions either by 5'-race PCR or by conventional hybridization screening techniques, followed by expression of the recombinant protein (Karlsen et al. (1991) Proc. Natl. Acad. Sci. USA 88:8337-8341; Karlsen et al. (1994) in: Insulin secretion and pancreatic beta-cell research, Flatt, P. R., et al., Smith-Gordon, USA; Chapter 64, pp. 1-9; Karlsen et al. (1995) Diabetes 44:757 758).

CRC-mediating proteins can be isolated in a variety of ways known to the skilled person in the art, including purification from biological material, and expression from recombinant DNA (see above). Conventional method steps include extraction, precipitation, chromatography, affinity chromatography, and electrophoresis. For example, cells expressing a CRC-mediating protein can be collected by centrifugation, or with suitable buffers, lysed, and the protein isolated by column chromatography, for example, on DEAE-cellulose, phosphocellulose, polyribocytidylic acid-agarose, hydroxyapatite or by electrophoresis or immunoprecipitation. CRC-mediating proteins may alternatively be isolated by immunoprecipitation with the use of specific antibodies.

Assay methods provided by the invention are useful for screening compounds capable of effecting the expression of a CRC-mediating protein, and thus the development of CRC in a mammal. One model for screening drugs capable of effecting the expression of one or more CRC-mediating proteins is the administration of compounds suspected of having beneficial effects (including antisense oligonucleotides) to cells in culture. Useful cells are inter alia colon or rectum derived cells. The effects of the test compound on protein expression may then be assayed by 2D gel electrophoresis.

Another screening model is an in vivo method with the use of a mammal at risk for development of CRC. Briefly, a mammal with an increased risk for CRC (e.g. CRC prone rat or mouse) is exposed to a test compound, and the effect of exposure to the test compound on the development of CRC is determined.

The development of CRC may be monitored throughout the developmental period by determining the expression of one or more CRC-mediating proteins and comparing by comparing the time of disease onset with expression and timing in the absence of disease development. Determining the expression of one or more CRC-mediating proteins includes the CRC-mediating protein itself, a post-translational modification product, and/or a CRC-mediating protein degradation product. In one embodiment, activation of a CRC-mediating protein is determined by measuring the level of the CRC-mediating protein expression in a test sample. A suitable test sample includes a body fluid, such as blood, urine, feces, colorectal tissue, or cerebrospinal fluid, or fluid derived from it, such as plasma or serum. In a specific embodiment, the level of protein expression in a test sample is measured by Western blot analysis. The proteins present in a sample are fractionated by gel electrophoresis, transferred to a membrane, and probed with labeled antibodies specific for the protein(s).

In another specific embodiment, the level of CRC-mediating protein expression is measured by Northern blot analysis. Polyadenylated [poly(A)+] mRNA is isolated from a test sample. The mRNA is fractionated by electrophoresis and transferred to a membrane. The membrane is probed with labeled cDNA. In another embodiment, protein expression is measured by quantitative PCR applied to expressed mRNA.

In yet another aspect, the invention provides for methods for identifying compounds capable of suppressing or reducing the expression of an endogenous deleterious protein, as well as methods for preventing and/or treating CRC by administering a therapeutically effective amount of a compound capable of suppressing or reducing the expression of an endogenous deleterious protein.

The CRC-mediating proteins of the invention are also useful to screen reagents that modulate CRC-mediating protein activity. Accordingly, in one aspect, the invention features methods for identifying a reagent which modulates CRC-mediating protein activity, by incubating a cell expressing a CRC-mediating protein with the test reagent and measuring the effect of the test reagent on CRC-mediating protein synthesis, phosphorylation, function, or activity. When activation of a CRC-mediating protein is via phosphorylation, the test reagent is incubated with the CRC-mediating protein and with either gamma-[³²P]; or [³³P]-labeled-ATP (or other mono-nucleotides), or [³²P]; or [³³P]-pyrophosphate (phosphoric acid) or [³⁵S]-methionine, and the rate of phosphorylation determined.

In another embodiment, the test reagent is incubated with a cell transfected with a CRC-mediating protein polynucleotide expression vector, and the effect of the test reagent on CRC-mediating protein transcription is measured by Northern blot analysis.

In a further embodiment, the effect of the test reagent on CRC-mediating protein synthesis is measured by Western blot analysis using an antibody to the CRC-mediating protein.

In still another embodiment, the effect of a reagent on CRC-mediating protein activity is measured by incubating CRC-mediating protein with the test reagent, [³²P]-ATP (or other radiochemicals mentioned above), and a substrate in the CRC-mediating protein pathway. All experiments would be compared against a normal labelling of cells with [³⁵S]-methionine to determine modulation of protein expression. The rate of substrate phosphorylation is determined by methods known in the art. The term modulation of CRC-mediating protein activity includes agonists and antagonists. The invention is particularly useful for screening reagents that inhibit deleterious protein activity. Such reagents are useful for the treatment or prevention of CRC.

The invention provides methods for preventing and/or treating CRC in a human by administering a therapeutically effective amount of a protective CRC-mediating protein. Preferably the mammal is a human subject at risk for CRC.

In a drug-screening assay of the invention, identified protective or deleterious CRC mediating proteins are used to identify test compounds capable of effecting their expression. Test compounds so identified are candidate therapeutic agents for preventing, ameliorating, or delaying the onset of CRC in a subject at risk.

A test therapeutic compound which effects the expression of a CRC-mediating protein can be, but is not limited to, at least one selected from a nucleic acid, a compound, a protein, an element, a lipid, an antibody, a saccharine, an isotope, a carbohydrate, an imaging agent, a lipoprotein, a glycoprotein, an enzyme, a detectable probe, and antibody or fragment thereof, or any combination thereof, which can be detectably labeled as for labeling antibodies, as described herein. Such labels include, but are not limited to, enzymatic labels, radioisotope or radioactive compounds or elements, fluorescent compounds or metals, chemiluminescent compounds and bioluminescent compounds.

A therapeutic compound is identified in the drug screening assay of the invention through its ability to induce or enhance the expression of a protective protein, such that disease onset is prevented or delayed in a subject at risk for the development of CRC. A candidate therapeutic compound is also identified by its ability to prevent or decrease the expression of a deleterious protein, such that disease onset is prevented or delayed in a subject at risk for the development of CRC.

A therapeutic nucleic acid as a therapeutic compound can have, but is not limited to, at least one of the following therapeutic effects on a target cell: inhibiting transcription of a deleterious protein DNA sequence; inhibiting translation of a deleterious protein RNA sequence; inhibiting reverse transcription of an RNA or DNA sequence corresponding to a deleterious protein; inhibiting a post-translational modification of a protein; inducing transcription of a DNA sequence corresponding to a protective protein; inducing translation of an RNA sequence corresponding to a protective protein; inducing reverse transcription of an RNA or DNA sequence corresponding to a protective protein; translation of the nucleic acid as a protein or enzyme; and incorporating the nucleic acid into a chromosome of a target cell for constitutive or transient expression of the therapeutic nucleic acid. Therapeutic effects of therapeutic nucleic acids can include, but are not limited to: turning off a defective gene or processing the expression thereof, such as antisense RNA or DNA; inhibiting viral replication or synthesis; gene therapy as expressing a heterologous nucleic acid encoding a therapeutic protein or correcting a defective protein; modifying a defective or under-expression of an RNA such as an hnRNA, an mRNA, a tRNA, or an rRNA; encoding a drug or prodrug, or an enzyme that generates a compound as a drug or prodrug in pathological or normal cells expressing the CRC-mediating protein or peptide; and any other known therapeutic effects. Also included in the invention is gene therapy by providing a polynucleotide encoding a protective CRC-mediating protein. The invention further includes a method for preventing CRC by administering an effective amount of a polynucleotide which inhibits the in vivo expression of a deleterious CRC-mediating protein.

In the therapeutic method of the invention, a therapeutic compound is administered to a human patient chronically or acutely. Optionally, a protective protein is administered chronically in combination with an effective amount of a compound that acts on a different pathway than the therapeutic compound.

The therapeutic method of the invention can be combined with other treatments for CRC or with methods for the management of this type of cancer.

As described in the examples below, human epithelial cells derived from the colon of patients suffering from CRC and healthy control persons were cultured under standard culture conditions.

After incubation for 20 hr under standard cell culture conditions, cells were labelled in the presence of [³⁵S]-methionine for 4 hours. Proteins were isolated from both cells and culture media. Protein samples were analyzed by 2-D gel electrophoresis and mass spectroscopy.

For analytical gels, used for the identification of proteins of altered expression level, individual experiments were performed with colon epithelial cells in each experiment. This allowed the construction of a composite image of each culture condition. Thus, comparison and statistical evaluation between the control and the cancer cells by this means allowed identification of a set of proteins in the cancer cells as well as secreted to the medium that were significantly up- or down-regulated compared to the control situation.

### EXPERIMENTAL

The following examples are put forth so as to provide those of ordinary skill in the art with a complete disclosure and description of how to make and use the proteins, genes and assays of the present invention, and are not intended to limit the scope of what the inventors regard as their invention. Efforts have been made to ensure accuracy with respect to numbers used (e.g. amounts, temperature, etc.) but some experimental errors and deviations should be accounted for.

Unless indicated otherwise, parts are parts by weight, molecular weight is weight average molecular weight, temperature is in degrees Centigrade, and pressure is at or near atmospheric.

### EXAMPLE - CHARACTERIZATION OF COLORECTAL CANCER-MEDIATED PROTEINS

### Reagents

Chemicals used for lysis buffer and equilibration buffer were: urea (ICN Biomedicals), thiourea (Fluka), chaps (Sigma), DTT (Sigma), SDS (Serva). Dulbecco's Modified Eaglr Media (D-MEM) (41966-029/052) and Hank's Balanced Salt Solutions (HBSS) (14175-053) were from GIBCO^{™}. RNA'se A (cat.no.RAF LS005650) and DNA's I (cat.no.DPFF LS006330) for DNA'se/RNA'se buffer were from Worthington. Scintillation liquid for determination of isotope incorporation was from Canberra Packard. Immobilized pH-gradient strips pH 4-7, pharmalytes 3-10 (Code No. 17-0456-01), IPG buffer 6-11 (Code No. 17-6001-78) and [³⁵S]-methionine were from Amersham Pharmacia Biotech. Acrylamide used for second dimension gels was from Genomic Solutions. Dye reagent (Bio-Rad protein assay Catalog No 500-50006) used for protein determination and N, N'-Methylene-bis-acrylamide were from Bio Rad. Colorectal normal/cancer tissues were obtained from Vejle Hospital as part of their normal surgical procedures for therapy.

### Material collection

immediately after surgical removal, the specimen is transported to the pathological department. There the specimen was opened and samples were taken from tumor/normal mucoma and transferred to different tubes containing transport medium (D-MEM which have dialyzed human serum, 1g Glucose/L and antibiotic penicillin/streptomycin in but without Methionine, keep in 37°C).

### Biopsies labeling

The human colon biopsies (normal/cancer) were transported from Vejle Hospital in transport medium at 37°C and rinsed 3 times using HBSS. The biopsy was then dissected out in 1mm² and some were removed to the labeling medium (D-MEM have dialyzed human serum, 1g Glucose/L, [³⁵S]-methionine ). The left over of the biopsy is put into the liquid nitrogen and stored in -80°C. After labeling for 24hours at 37°C, the used radioactive medium was harvested from each well transferring the used medium to a marked 1.5mL Eppendorf tube. The medium is then incubated in the incubator at 37°C. The biopsies were washed twice in HBSS; the washing solutions then were collected for centrifugation to harvest the loose cells and tissues. After that, the HBSS were removed and the biopsies are transferred to an empty and dry well in the tray, and transferred again to an ice cold homogenizer.

### Protein extraction

100µl DNA'se/RNA'se buffer (25µg/ml RNAse A solution, 25ug/ml DNAse I solution, 30mM NaCl, 20mM Tris.HCl (pH7.5), 5mM CaCl₂, 5mM MgCl₂) was added to the ice cold homogenizer containing the biopsies and homogenized for 30seconds. After 5 to 10min they were homogenized again. This was repeated for 30min until all the tissues were homogenized. Remove 150µl homogenate to a clean tube. Use 100ul H₂O wash the homogenizer and the pipette twice and the H₂O was also removed to the tube. The tube was them put into the liquid nitrogen and filled with nitrogen. Thereafter the tube was put into the freeze dryer overnight. 300µl lysis buffer (7M Urea, 2M Thiourea, 2% CHAPS, 0.4% DTT, 1% v/v pharmolite 3-10, IPG buffer 6-11) was added to the tube and shaken at room temperature overnight.

### Protein and CPM determination

Bradford method (*Bradford MM. 1976*) adapted for use with lysis buffer was used to determine the protein concentration in the samples. In triplicate, a standard curve was prepared by adding 10µl lysis buffer to a series of disposable plastic cuvettes which contain 0, 5, 10, 20 and 40µg standard protein (BSA). H₂O was added to each cuvette till a total volume of 1600µl. 10µl of each sample were added in triplicate to 1590µl H₂O in cuvettes. 400µl dye reagent was then added to each cuvette and mixed thoroughly. The absorbance at 595nm of each sample was measured within 30 minutes of adding the dye reagent. Protein concentrations were then determined by the standard curve. (*Fey SJ, et al*., 1997)

The isotope incorporation into the protein was determined using the method as follows. 5µl supernatant of each sample was taken in duplicate and added to 45µl of 0.02% BSA. After mixing them in a micro-test tube, 1ml 10% w/v TCA was added and left for 30min at 4°C to precipitate the protein. The precipitate was then collected by suction filtration using HAWP filters (HAWP 0200, Millipore, Bedford, MA, USA) which were pre-wetted with 1%BSA in 10% w/v TCA. The filters were dried with a hair drier and transferred to scintillation vials. 4ml Scintillation liquid were then added and left for at least 2h. Then the samples were counted using scintillation counter (Canberra Packard 2000). (*Fey SJ, et al., 1997*)

### Gel electrophoresis

The proteins were separated in the first dimension on 18cm IPG4-7 strips. The rehydration buffer used for IPG4-7 strips was the lysis buffer. All samples were added to 200µl and this was applied to the IPG strips and left for 18hours to reswell. After this a further 100µl of lysis was added and to wash the sample in, left for 5.5hours.(*Adelina Rogowska-Wrzesinka, et al., 2001*) 5x 10⁶ CPM were loaded on each gel.

Focusing was carried out on Multiphor II using a total Vh 50,000 (linear increasing gradient from 0V to 600V for 2:15h, 600V to 3500V for 8h and then constant at 3500V for 9:25h) at 20°C. As soon as the focusing was finished, the strips were shaken for 15min in equilibration buffer (6M Urea, 30%Glycerol, 2% SDS, 1%DTT, 50mM Tris-HCl pH 8.8) and stored at -80°C.Prior to loading onto the second dimension; the strips were equilibrated for another 15min in fresh equilibration buffer.

In the second dimension, SDS PAGE gel electrophoresis was performed using 12.5% w/v acrylamide gels (acrylamide: bisacrylamide ratio 200:1) on a vertical Investigator 2-D Electrophoresis System (Millipore). The gels were run at 20°C overnight at a constant current setting. The running buffer was recirculated.

### Protein pattern visualization and computer analysis

After the two dimension gel electrophoresis separation, the gels were dried directly on filter paper, exposed to phosphoimager plates (AGFA) for 10days, and read in the AGFA ADC70 reader (Morstel, Belgium). The gels were then marked in the corners with radioactive ink and exposed to film (AGFA,Curix RP2) for 25days in order to prepare cutting masks and protein identification.( *Nawrocki A, et al., 2001)*

The 2D gel patterns obtained from the ADC70 phosphoimager were analyzed using Bioimage 2-D Analyzer (UNIX based). Spot boundaries were found using the same parameters for all gels. In some cases boundaries were corrected manually (e.g. to divided spots that were too close for the program to differentiate. Protein expression is measured as the sum of all the pixel values within the spot boundary (integrated optical density, IOD). (*Nawrocki A, et al., 2001*) This is given as a percentage of the total of all the spots in one gel (%IOD). (*Adelina Rogowska-Wrzesinka, et al., 2001*) All the images were compared with the reference image (comes from previous analysis). The spots which present in more than 5 images and miss in Ref. image have been added to Ref. image on the correct positions.

The match number and %IOD of each spots were extracted and all the data were put together with the previous data. The data were divided into two groups: normal cells and cancer cells. The standard deviation for each spot was calculated in each group and the comparison results of the square standard deviation between normal and cancer group for each spot was used for the next student's *t*-test.

The student's *t*-test between the two groups was calculated for each spots to reveal proteins whose expression was significantly different. (Significance level 99%, p=0.01) The proteins which were significantly different were then selected.

### Preparative gels

For the following protein identification by mass spectrometry, Preparative IPG4-7 gels were run by loading 300µg/gel cold proteins in addition to 3×10⁶ CPM/gel [³⁵S]-methionine labeled proteins using the same gradients and procedures. After the second dimension, the preparative gels were dried and exposed to X-ray film for 10days. (*Adelina Rogowska-Wrzesinka, et al., 2001*)

### Protein Identification

The developed X-ray film was put on the dried gel, using the marked on the corner to locate the proteins of interest. A transparency film was located to relocate the proteins nad using as a cutting mask when the X-ray film was removed. The proteins were then excised directly from the gel. The remaining white filter paper was removed from the back of the gel by washing in H₂O and dried by vacuum centrifugation until it became white on the surface. Then the gel was allowed to reswell in 50mM NH₄HCO₃, 5mM CaCl₂, 12.5ng/µL trypsin at 4°C for 45min. The supernatant then was replaced by 5-10µL same buffer without trypsin and left to digestion overnight at 37°C (*Fey SJ, et al., 1997*). After in gel digestion, they were analyzed by MALDI mass spectrometry. (*Jensen ON, et al., 1998*) The mass spectra obtained were internally calibrated using trypsin autodigestion peptides (*Fey SJ, et al., 1997*). Then they were used to search the NCBI database via MASCOT MS/MS ion search (http://www.matrixscience.com).

The following parameters were used for the database search with minor modification: mammals, trypsin digestion, oxidation of methionines possible, one missed cleavage allowed, no protein mass and pl restrictions, peptides tolerance ± 50-70ppm, MS/MS tolerance ± 0.5Da, peptide charge +1, Monoisotopic, MALDI-TOF-TOF is the method used.

A 'significant' result can be considered as identification combined with search the SWISS-PROT database and review the protein position in the 2D gel pattern. Protein function was cited from the NCBI database (http://www.ncbi.nlm.nih.gov/entrez/query.fcgi?db=Protein), SWISS-PROT database (http://www.ncbi.nlm.nih.gov/entrez/guery.fcgi?db=Protein) and other publication which is referred to.

### Results

In this study, normal and cancer colon biopsies from colon cancer patients were labeled and analyzed by 2DGE after protein extraction. Normal /cancer biopsies were got from 33 patients and totally 65 samples were used (one cancer sample was contaminated with bacteria). The proteome of each sample was analyzed using IPG 4-7 gels.

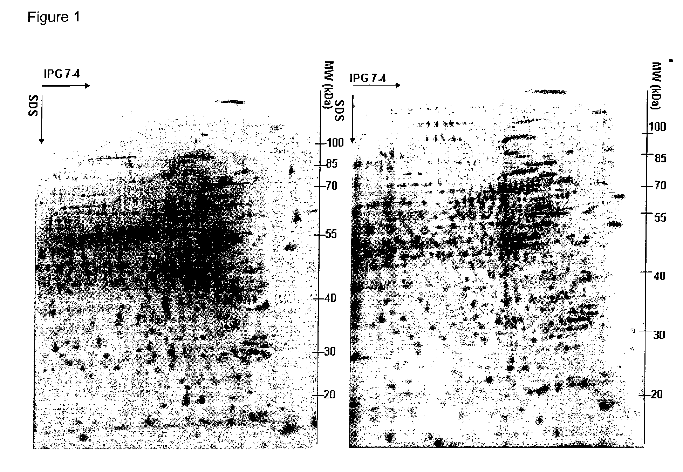

Using computer-aided image analysis, 2,128 spots were detected and matched. The [³⁵S]-methionine labeled proteins 2DGE patterns were then divided into two groups: normal and cancer. There are 33 patterns in 'Normal' group and 32 patterns in colorectal cancer biopsies group. Examples of the gel images are shown in Figure 1.

Figure 1 shows 2DGE pattern of [³⁵S]-methionine labeled proteins from normal human colon tissue (left) and colorectal cancer biopsies. The normal tissues are from a 65 years old female and the colorectal cancer biopsies are from a 59 years old male.

Comparison and statistical analysis between these two groups were carried out thereafter. 298 protein spots were shown to be significantly changed between the two groups. They were all cut from the IPG 4-7 preparative gels and subjected to MALDI mass spectrometry. To date 66 spots have been identified and more are on the way.

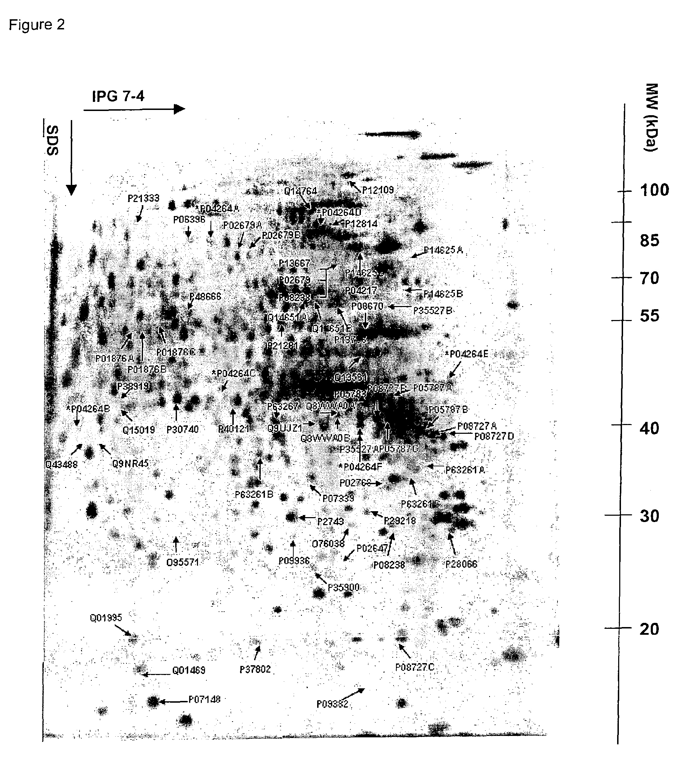

Among these spots, 25 spots are up-regulated in cancer and 41 spots are down-regulated in cancer. 31 spots are of low intensity (average %IOD< 0.05 in both normal and cancer group). That is 47% of the total spots identified. It shows that many weak spots can be identified. The positions of the identified proteins were shown in the 2DGE pattern in Figure 2 and the quantitation of these proteins is given in Table 1.

Figure 2 shows a 2DGE pattern of [³⁵S]-methionine labeled proteins from normal human colon biopsies. IPG 4 - 7 gradient was used for protein separation in the first dimension and SDS-PAGE in 12.5% polyacrylamide were used in the second dimension. All the names are the identified proteins' Accession number in Swiss-prot database. Some are possible post translational modification proteins, some may be larger proteins and their breakdown fragments or post translational modification proteins, some are Proteins that has been reported to have different expression in human colorectal cancer or have been considered as a possible diagnostic marker for colorectal cancer. The 'A', 'B', 'C'... after the proteins' Accession number correlates to the 'A', 'B', 'C'... in Table 1 for different %IOD

### Possible post translational modification

Ten proteins identified were found in more than two spots. Among them, Keratin 1 (marked as * in Figure 2) is identified as six spots placed quite distant to each other. Keratin 1 is always considered as contaminants during the mass spectrometry. So these spots need to be analyzed again.

Six of them were identified as two or have two in-row spots quite close to each other. This kind of spot pattern is considered as a type of post translational modification that changes the pl of the protein but without a big change of the molecular weight.

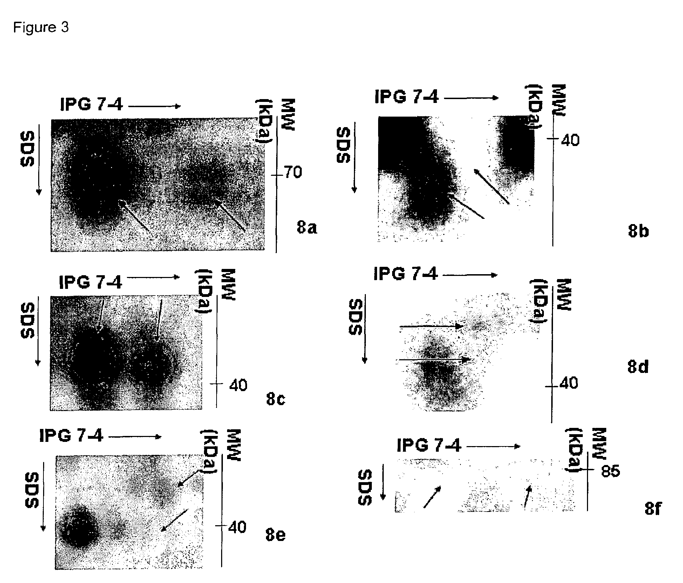

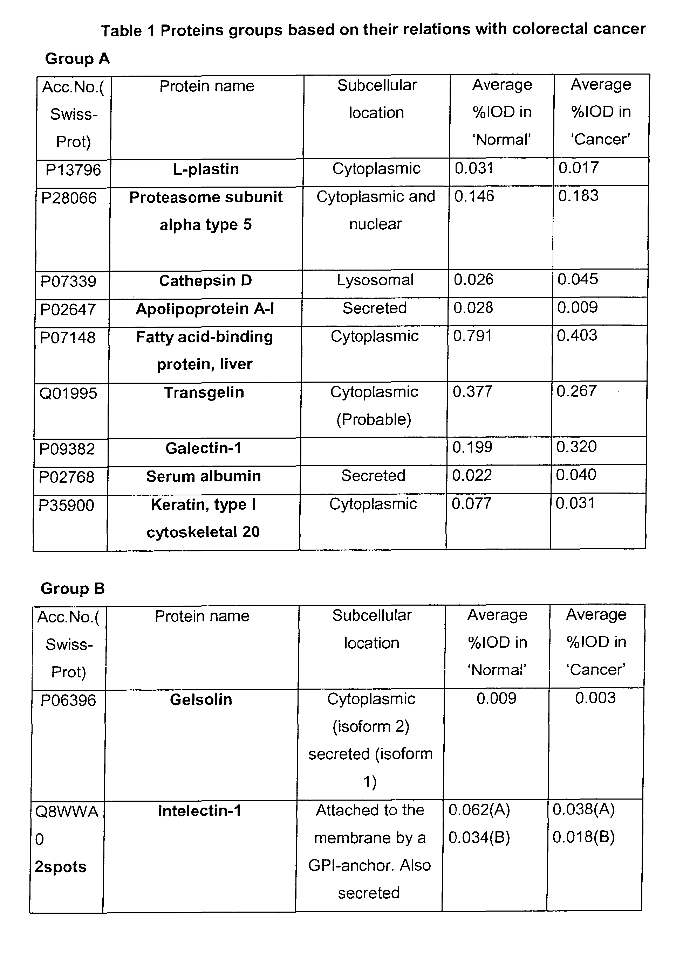

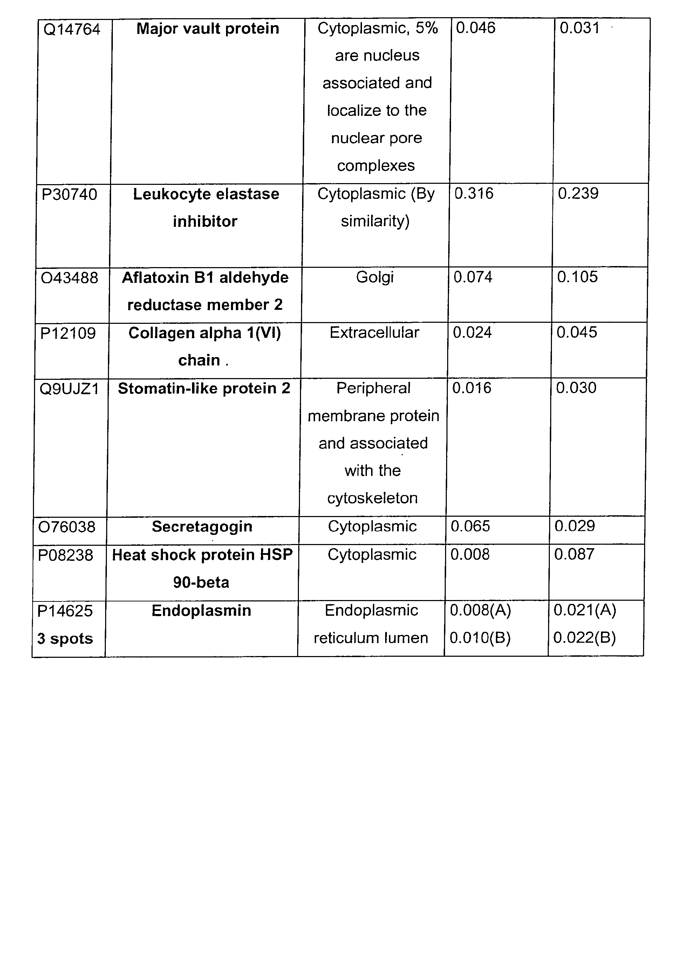

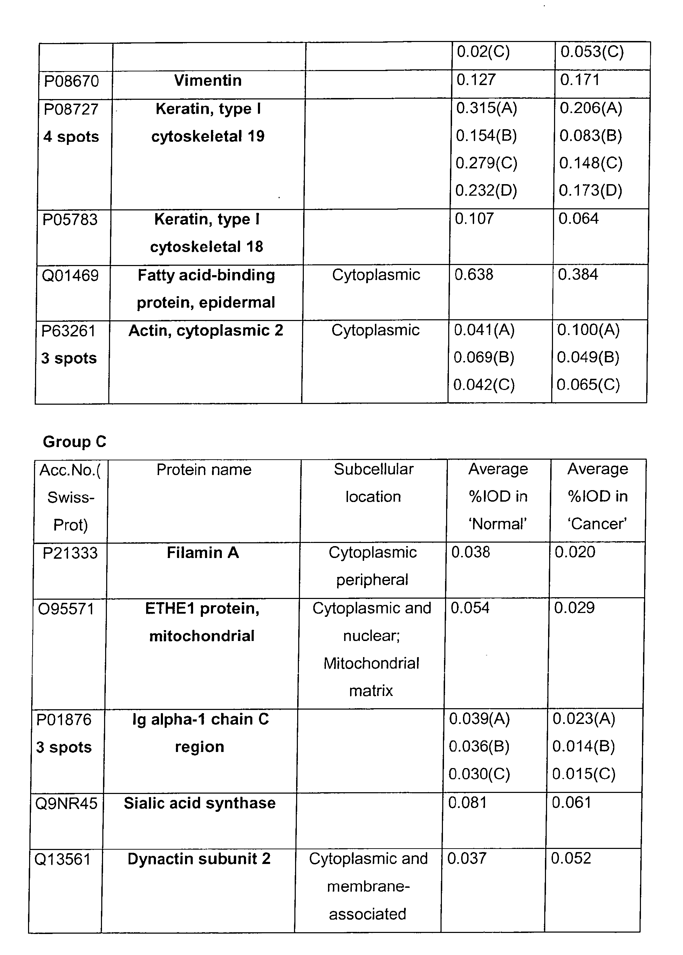

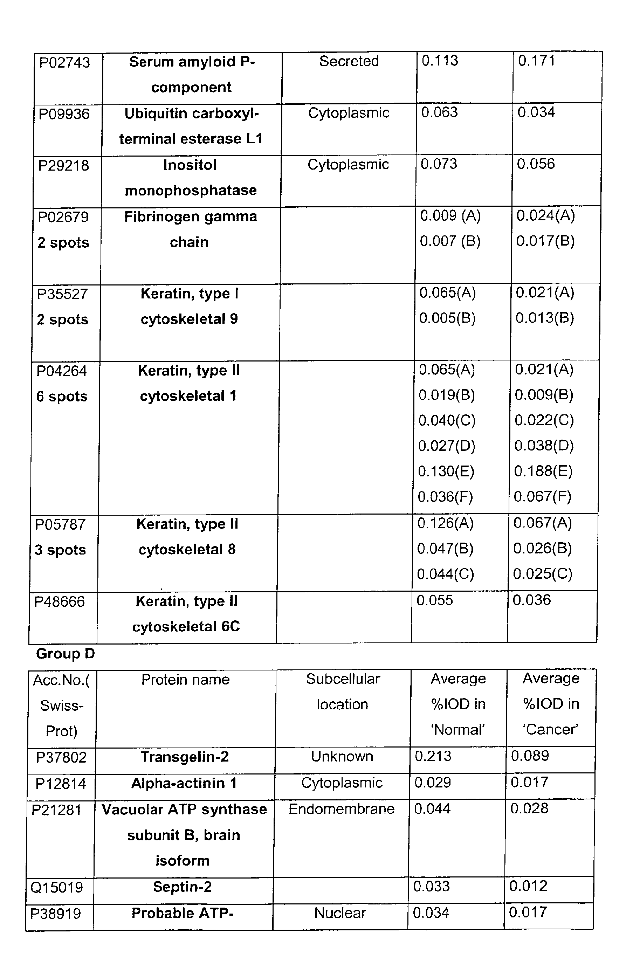

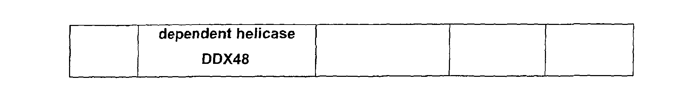

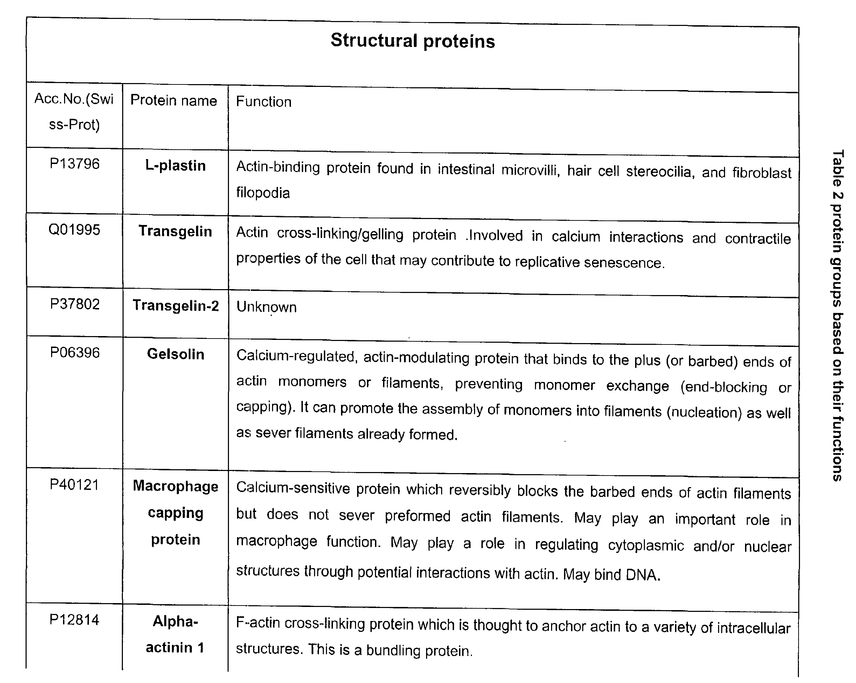

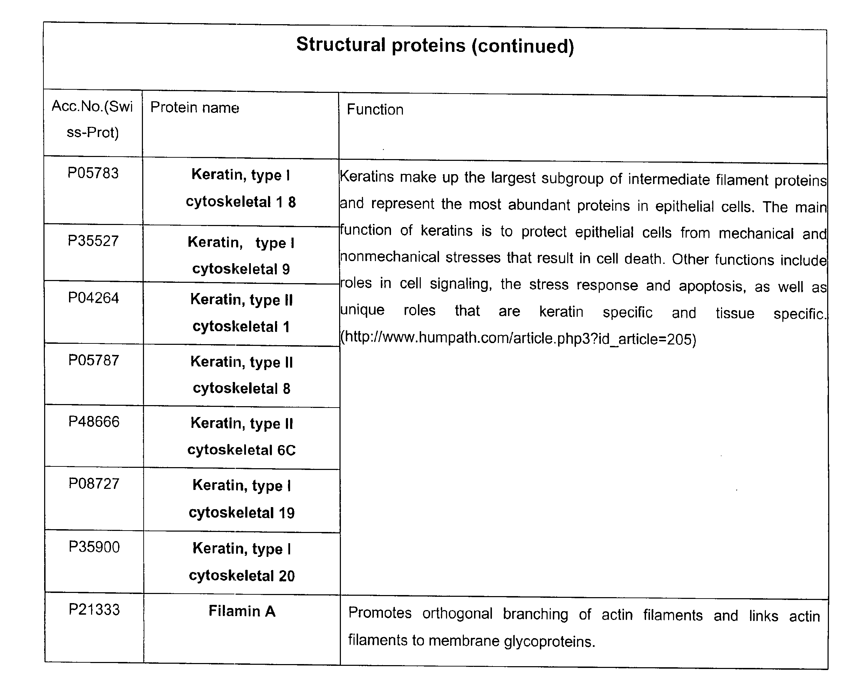

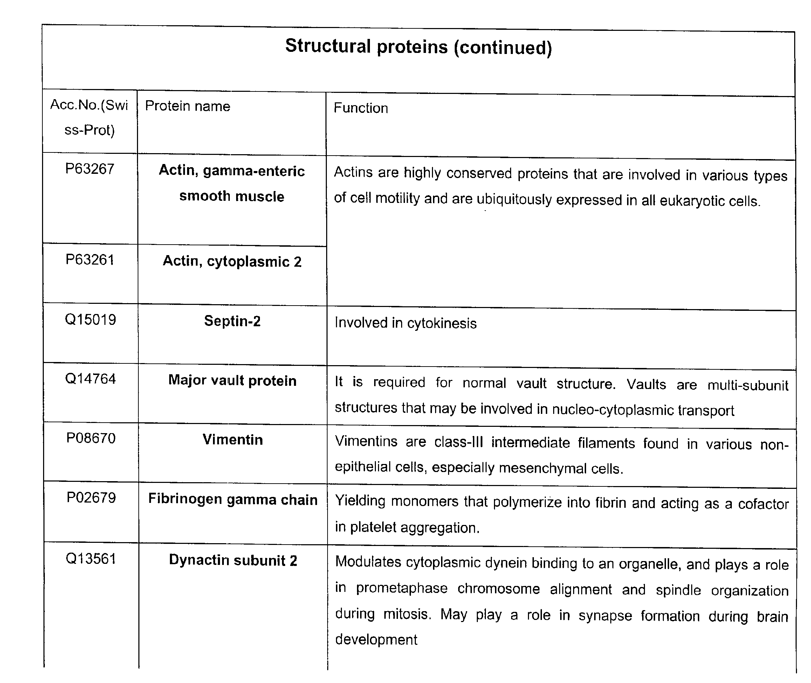

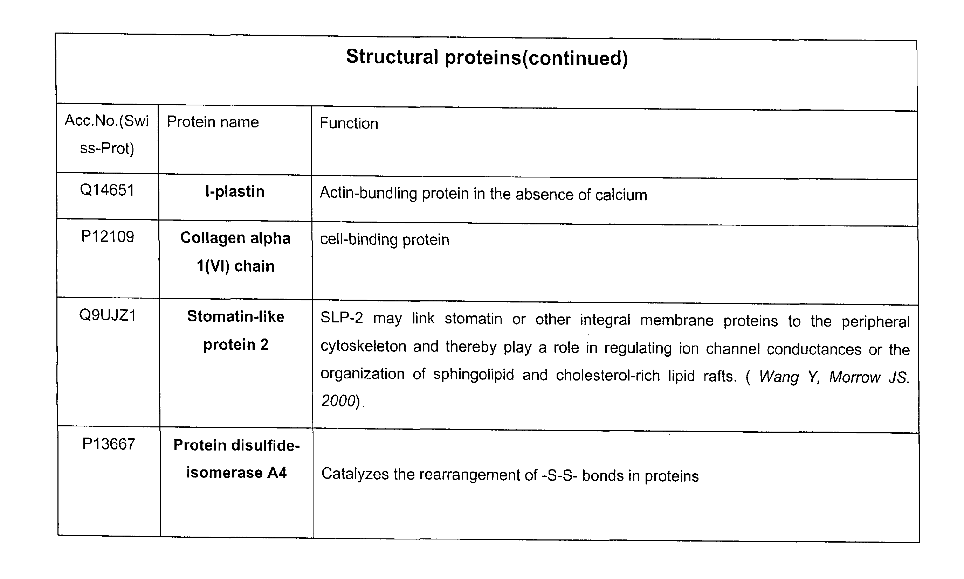

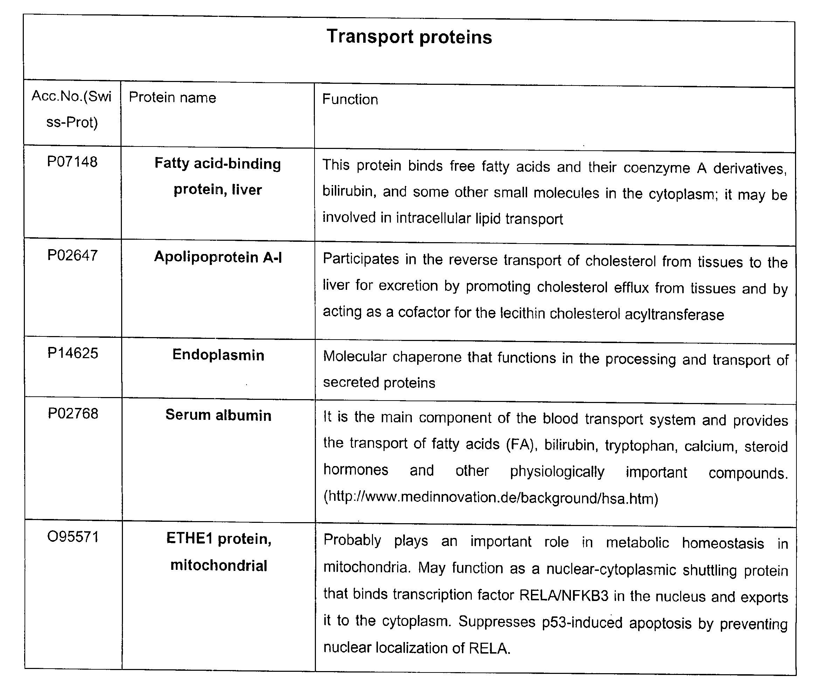

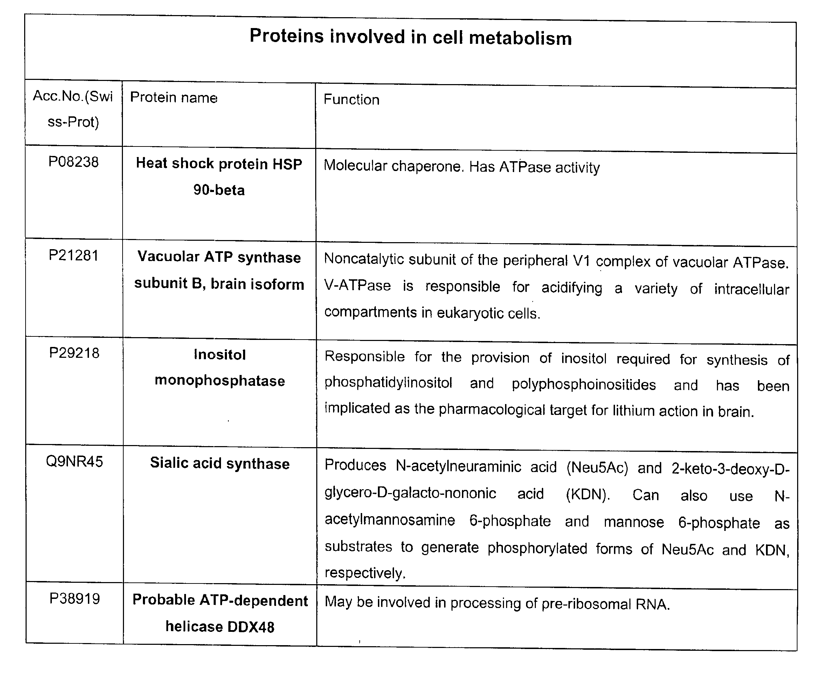

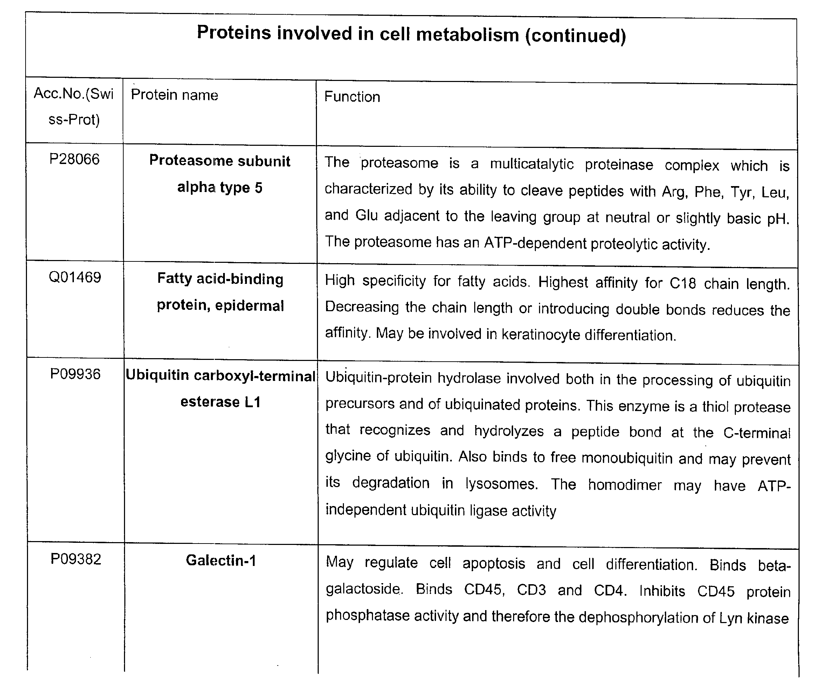

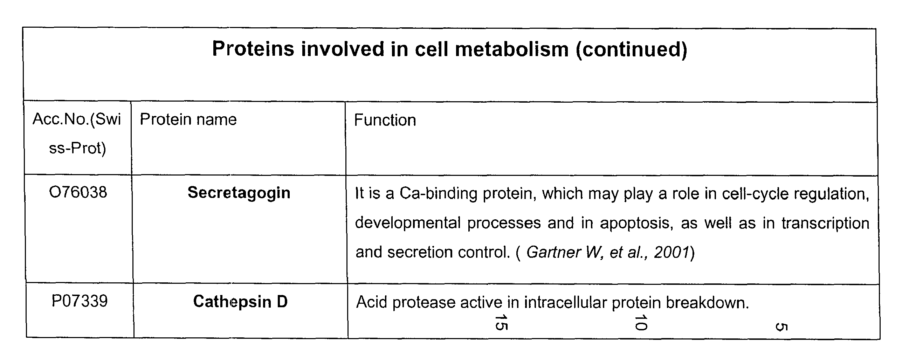

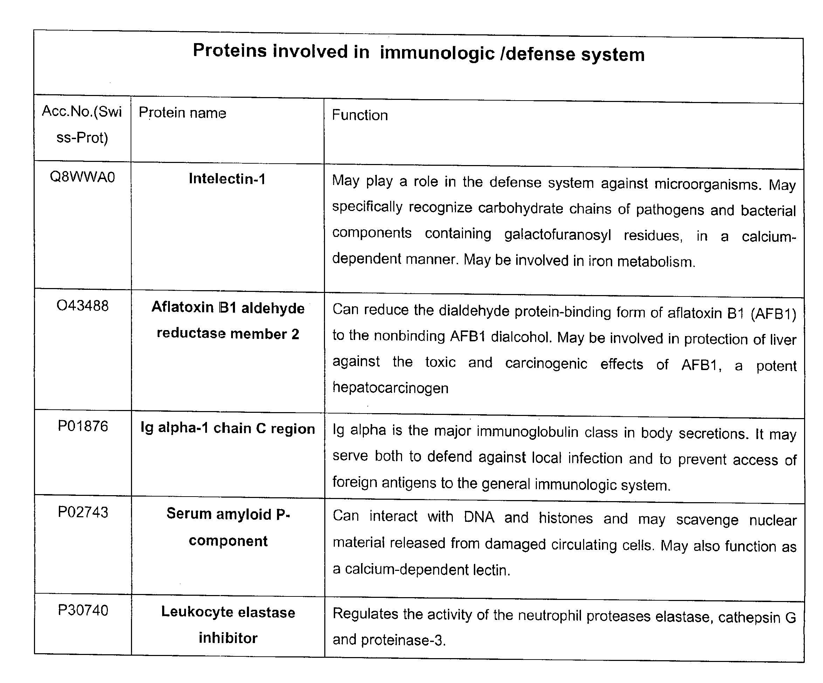

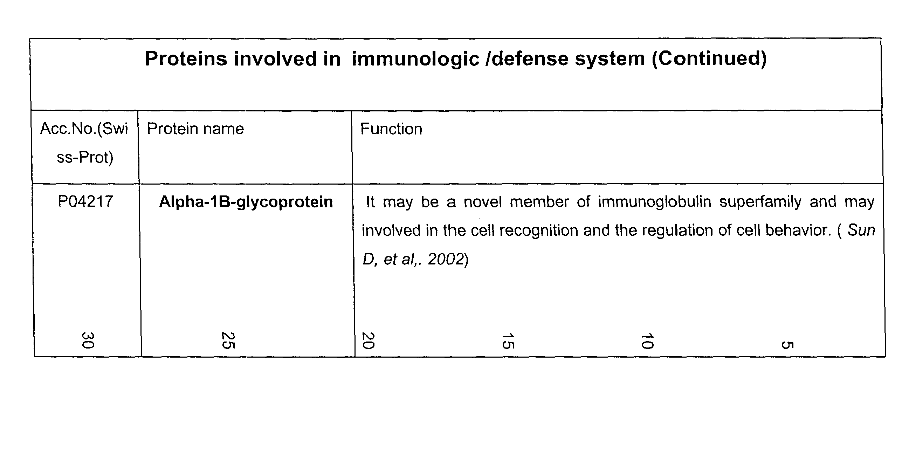

Figure 3 shows enlarged 2DGE patterns of the possible PTM spots: I-plastin (9a); intelectin (9b); Keratin 19 (9c); Ig alpha chain C region (9d); Actin, cytoplasmic 2 (9e); Fibrinogen gamma chain precursor (9f)

For example, I-plastin(Q14651) is identified as two spots and both down-regulated in colorectal cancer biopsies. The more neutral one is more abundant than the more acidic one. Phosphorylation is the predicted PTM and we can confirm the conclusion by the spots positions in 2DGE pattern. (Figure 2 and 3a, Table 1).

Intelectin-1 (Q8WWA0) is identified as two spots and both down-regulated in colorectal cancer biopsies. N-glycosylation is the identified PTM (Swiss-prot). (Figure 2 and 3b) Keratin, type I cytoskeletal 19 (P08727) is identified as four spots and all of them are down-regulated in colorectal cancer biopsies. Two of the spots are quite close (Figure 2 and 3c). Keratins are known to be phosphorylated, so phosphorylation could be the predicted PTM.

Ig alpha-1 chain C region (P01876) is identified as three spots which are all down-regulated in colorectal cancer biopsies. Two of the spots are quite close (Figure 2 and 3d). Cys-352 is the proteins modification site which forms non-reducible thioether cross-links after the reaction between chromophore and accessible cysteines.

Actin, cytoplasmic 2 (P63261) is identified as three spots. Two of them which are very close to each other (Figure 2 and 3e) are up-regulated in colorectal cancer biopsies and another one is down-regulated in colorectal cancer biopsies.

Fibrinogen gamma chain precursor (P02679) is identified as two spots and both up-regulated in colorectal cancer biopsies. The sulfation of C-terminal tyrosines has been predicted as PTM which can increase affinity for thrombin, the enzyme that triggers the conversion of fibrinogen to fibrin. (Figure 2 and 3f).

Five of the proteins are identified as two or three spots which are located in relative close positions (marked in Figure 2). They may be larger proteins and their breakdown fragments. Because of the rapid sample preparation procedures used here, we consider these breakdown fragments to be produced in the living tissue and therefore significance.

Three of them (P14625, P05787, P08727, P63261) can also be considered as post translational modification that changes the pl of the proteins but without a big change of the molecular weights.

P08727 and P63261 have been discussed above. Endoplasmin precursor (P14625) was identified as two up-regulated and one down-regulated spots in colorectal cancer biopsies (Figure 2), while the down-regulated one was identified in protein mixture Spot C, so the expression of the proteins in colorectal cancer biopsies can probably be up-regulated. No protein modification has been reported of this protein.

Keratin, type II cytoskeletal 8 (P05787) was identified as three spots which close to each other and all the three spots are down-regulated in colorectal cancer biopsies. (Figure 2) Phosphorylation of Ser-73 plays an important role in keratin filament reorganization.

### Proteins Mixtures

Three of the spots were identified as a mixture of two or three proteins.
Spot A: P35527 and P04264 (sequence coverage: 32% and 22%).
Spot B: P13667, P02679 and P08238 (sequence coverage: 23%, 33% and 24%);
Spot C: P14625 and P04217 (sequence coverage: 17% and 10%); (Figure 2)
Usually one of the components has a better coverage and a higher match score. The other components may be breakdown products of larger proteins, and the peptide masses cover fragments of the protein sequences (Spots B), or maybe they are both be breakdown products of larger proteins (Spot C), or maybe some contaminants, such as keratin, were involved in (spot A). These fragments cannot be generated during the sample preparation by random, because all the spots selected have a statistically significant change between the two groups which has more than 60 samples in all. In other words, these degradation products are specifically generated in the tissue or cancer biopsies.

### Protein functions

The identified proteins can be grouped by their different functions or pathways. All the data were obtained from Swiss-prot database if not specially mentioned. Among the 47 proteins, 25 proteins are structural proteins which is more than half (53%) of all the identified proteins. This is quite easily to be understood because during the tumor development, the normal structure of the cells could be destroyed or modified, for example, the cytoskeleton of the cell could be modified to influence the ion channels. Also, 23% (11) are proteins involved in cell metabolism. For example, galectin-1, secretagogin and cathepsin D play a role in apoptosis, cell differentiation or cell-cycle regulation. Their expression must be influenced once the tumor developed. 5 proteins are transport proteins, one explanation for this is by the change of the cell structure, for example, the membrane, the channels of some proteins' transportation in and out of cells are blocked and this influence the expression of the transport proteins. The reverse is the same. 4 proteins are involved in immunologic/defense system. It may because once the immunologic system recognized the abnormal cell activities, such as tumor development, more immunological cells infiltrate the colon and rectum to stop it. Some proteins like serum albumin which have multi-functions are put into the group that can represent its main functions. For further details see Table 2

### Proteins' relations with colorectal cancer

The identified proteins can also be divided into four groups on the basis of previous scientific reports:
- Proteins that have been reported to have different expression in human colorectal cancer compared with normal colorectal tissues or has been considered as a possible diagnostic marker for colorectal cancer. (Group A)
- Proteins that has been reported to have different expression in other cancers (i.e. breast cancer) compared with normal tissues or has been considered as a possible diagnostic marker. (Group B)
- Proteins that has been reported to have relationship with other diseases. (Group C)
- Proteins that have no reports about their relationship to any diseases. (Group D)
The protein names, subcellular locations, average %IODs in colorectal normal and cancer tissues are listed in table 1.

Group A (Proteins that have been reported to have different expression in human colorectal cancer compared with normal colorectal tissues or has been considered as a possible diagnostic marker for colorectal cancer) is then selected. The function and role in the development of colorectal cancer of each protein from this group will be further discussed. Among them, 3 are structural proteins, 3 are transport proteins, and 3 are proteins involved in cell metabolism. 5 proteins are down-regulated in colorectal cancer biopsies and 4 proteins are up-regulated.

### Down-regulated proteins

### L-plastin

L-plastin is one isoform of plastin (T-plastin is another isoform) - a family of human actin-binding proteins which are conserved in eukaryote evolution and abundantly expressed in all normal replicating mammalian cells. (*Lin CS, et al. 1993*) It is expressed in hemopoietic cells where it can be phosphoryand has been found to be synthesized in many types of malignant human cells of non-hemopoietic origin. Because of its high expression in human tumor cell of solid tissue, either L-plastin may play a role in tumororigenesis or L-plastin gene has been activated during the development of the tumor. (*Lin CS, et al. 1993*) Some studies has been done on human prostate, breast and other steroid-regulated tracts (*Lin CS, et al. 1993, Zheng J, et al. 1997*) and the results show the expression of L-palstin is up-regulated in carcinomas. It may be the hormone receptors which are induced to regulate the expression of L-plastin.

Another experiment has been carried out on colon carcinoma cell line SW480 and SW620 derived from a single patient. Result shows L-plastin is over expressed in advanced colorectal cancer compared to the earlier stage. (*Otsuka M, et al. 2001*) Unfortunately, no comparison between normal and cancer colorectal cells was done in that study.

Interestingly, in our study, the expression of L-plastin is down-regulated in cancer tissue compared to normal colon tissue. Because this protein has significant change between two groups which have more than 60 samples totally, it cannot be generated randomly. The average 'Cancer'/'Normal' ratio of %IOD is 0.55 which also cannot be ignored. The possible explanation for this is the expression of L-plastin in colon tissue is different from that in steroid-regulated tracts such as breast, prostate, ovary *et al*. One possibility is that it may be down-regulated from normal tissue to colorectal cancer biopsies and up-regulated from early cancer stage to advanced cancer stage. One explanation might be the brush boarder of the small intestine is normally growing-if that growth is faster than the early tumor, it could happen. But this needs to be demonstrated by further study on cancer tissue of different stages instead of cell lines.

### Transgelin

Transgelin is a shape-change sensitive actin gelling and transformation protein which is always found in fibroblasts and smooth muscle. (*Lawson D, et al., 1997*) It interacts directly with actin filaments and makes their rapid gelation in polymerization. The actin binding site or the functional gelation site is associated with a cluster of positively charged amino acid residues. The activity of transgelin is controlled by ionic strength. It totally loses its ability at high ionic strength. Its gelling activity is controlled at low ionic strength via the formation of dimers or oligomers. In the formation of actin gels, it plays a bundling or cross-linking role. Because transgelin plays such an important role in cytoskeletal organization and activation in normal cells, its down-regulated expression may be found in oncogenically transformed cells. In these cells, cytoskeletal reorganization and abnormal migrations are important characteristic of metastasis. (*Shapland C, et al., 1993*) It has been demonstrated by investigating the expression of fibroblast transgelin in many cell lines. (*Lawson D, et al., 1997*)

Some study has been done to investigate the expression of transgelin in both human colon tumor cell lines and patient- derived human colon and breast tumor tissues. The results showed the loss or reduction of transgelin expression in both cell lines and tissues. (*Shields JM, et al., 2002*) The loss of transgelin is regulated at the level of gene expression. Its down-regulation expression is caused directly by Ras activation. This is important because mutation in Ras genes exist in about 30% of all human cancers) Also, in some tumor cells, Ras-independent mechanisms have the same function. In another words, we can say that the down regulation of transgelin is associated with the temporal expression of oncogenic Ras. (*Shields JM, et al., 2002*) In our research data, transgelin is clearly down-regulated in tumor tissues compared to normal colorectal tissues. The average 'Cancer'/'Normal' ratio of %IOD is 0.71 and because of its high quantity showed on the 2DGE, it should be a good early diagnostic marker for human colorectal cancer , probably after the formation of cancers. Also, because of the exist of anti-transgelin, it is possible to be tested.

### Liver fatty acid - binding protein

Liver fatty acid binding protein is a member of the fatty acid binding group of low molecular weight proteins which involve in the intracellular transport of long-chain bioactive fatty acid. (*Lawrie LC, et al., 2004*) It is specially expressed in hepatocytes and enterocytes. (*Yamazaki T, et al*., *1999*)Because of its lipid-binding characteristic, it plays an important role in the solubilization and intracellular partition of fatty acid. It makes the cellular uptake and intracellular processing of fatty acid easier.( *Storch J, Thumser AE.* 2000) It is also involved in the fatty acid-mediated signal transduction pathways and gene expression regulation. So, it has a role in the control of some important physiological functions such as cell division, growth, differentiation, *et al,* (*Lawrie LC, et al., 2004*). During tumor development and progression, these functions are all less regulated and the fastest growth is shown by the human embryo during its normal development.

The study on the expression of liver fatty acid binding protein in colorectal cancer done by Lawrie LC has shown a consistent lost in colorectal cancer and its loss is an early stage event in the cancer development. (*Lawrie LC, et al., 2004*)

In another aspect, among the patients who have a liver metastasis derived from colorectal cancer, the survival years of the liver fatty acid binding protein-positive patients after hepatic resection is higher than liver fatty acid binding protein-negative ones. (*Yamazaki T, et al., 1999*) It appears that the down-regulated of liver fatty acid binding protein is related to the development of tumor.

Our research data shows exactly the same down-regulated expression tendency from normal colorectal tissue to colorectal cancer tissue. And the average 'Cancer'/'Normal' ratio of %IOD is 0.51. Also, the quantity of the protein showed on the 2DGE is fairly high. This makes it easier to be detected. So, liver fatty acid binding protein can be a very good prognostic marker of colorectal cancer.

### Keratin, type I cytoskeletal 20

Keratin 20 is a cytoskeletal protein in gastrointestinal epithelial cells (*Schwerer MJ, et al., 1996*) which was first detected in 1992. (*Moll R, et al., 1992*) It is among so-called 'soft keratins' which belong to intermediate filament family. Soft keratin contains more than 20 members and divides into two main groups: type I (K9-20, acidic), type II (K1-8, neutral to basic). (*Zhou Q, et al., 2003*)
In the keratin family, keratin 20 has an uncommon distribution and till now it is poorly investigated. (*Zhou Q, et al., 2003*) For example, human keratin 20 has some special characteristic:
■ Compared with other type I keratins, it has only 58% identical amino acids in the conserved α-helical 'rod' domain in common with Keratin 14 - the closest related keratin; (*Moll R, et al., 1993*)
■ Its distribution is almost entirely limited to gastric, intestinal epithelium, urothelium, bladder, Merkel cells et al; (*Moll R, et al., 1992*)
■ Its expression in the more differentiated enterocytes is located along the cryptvillus differentiation axis
■ It lacks immunological cross-reactivity to most anti-keratin antibodies. ). (*Zhou Q, et al., 2003)*
Its expression is increased with differentiation in highly differentiated tissue. Many studies have been done on the relationship between keratin 20 and cancers. It has been demonstrated that it is important for the characterization of primary and metastatic human adenocarcinomas. (*Wildi S, et al*., *1999*) Also, it is considered as an adjunct marker to distinguish the primary origins of carcinomas, especially to separate adenocarcinomas of gastrointestinal and non gastrointestinal origin. (*Miettinen M, 1995*) Some other studies done on colorectal cancer cell lines showed the CK 20 mRNA was decreased in the colorectal cancer samples compared with normal colon samples. (*Wildi S, et al., 1999*) Keratin 20 was also been considered as a marker for submicroscopic lymph node metastases in colorectal cancer and validated by K-RAS, a gene mutated in about 30-40% of colorectal cancers. (*Yun K, et al., 2000*)

In our study, Keratin 20 is down-regulated in tumor tissues compared to normal colorectal tissues with a average 'Cancer'/'Normal' ratio of %IOD 0.4. However, the average quantity of the protein detected is relatively low, and it is difficult to use a protein that disappears for diagnosis, therefore it has to be used to differentiate tumor types and it might be as an adjunct diagnostic marker for human colorectal cancer.

### Apolipoprotein A-I

Apolipoprotein A-I is the major structural protein of High Density Lipoprotein (HDL) which have 234 residues. It belongs to the class of exchangeable apolipoprotein and contains a globular amino- terminal domain and a lipid- binding carboxyl- terminal domain. (*Segrest JP, et al., 2000; Marcel YL, et al., 2003*)

Its lipid binding characteristic helps make a delicate balance between the ability of the lipoprotein to dissociate into its constituents and the stable transport of lipids in the circulation. (*Marcel YL, et al., 2003*) It promotes cholesterol efflux from cells and this mechanism may play an important role in supporting cellular cholesterol homeostasis. (*Segrest JP, et al., 2000*) Also, its ability to bind lipids, activate lecithin, form mature HDL which interact with specific receptors and lipid transfer proteins can influence the efficiency of reverse cholesterol transport. And together with HDL, their roles in reverse cholesterol transport determined the inverse correlation between HDL plasma level and coronary heart disease. (*Frank PG, et al., 2000*)

Besides coronary heart disease, Apolipoprotein A-I has been reported to be important in other diseases such as rheumatoid arthritis, multiple sclerosis, systemic lupus erythematosus, and atherosclerosis, et al.( *Hyka N, et al., 2001*)

It has been shown that Apolipoprotein A-I is expressed in human intestinal epithelial cells. One study carried out by *Normanno* showed that Apolipoprotein A-I mRNA cannot be detected in healthy persons or patients without colon cancer using Apo A-I RT-PCR assay (4 healthy donors and 11 individuals with tumor disease other than colon cancer), while a low number of colon carcinoma patients were found to be positive. (*Normanno N, et al., 1998*) In our analysis result, the average quantity of the protein detected is quite low in both colorectal cancer biopsies and 'normal' group, though average 'Cancer'/'Normal' ratio of %IOD is quite good (0.32). This can be an explanation why *Normanno* could not detect it in his normal samples. Also the number of samples in *Normanno N'*s study is relatively small and the two positive results out of twenty can be considered as chance) One hypothesis for its down-regulation in colorectal cancer is that during the development of cancer the loss of Apolipoprotein A-I may happen to destroy the homeostasis of cholesterol efflux, disturbing the cellular metabolism. But such assumption needs to be confirmed by later study.

### Up-regulated proteins

### Cathepsin D

Cathepsin D is a member of cathepsin family which has a role in the degradation of the extracellular matrix. (*Adenis A, et al., 1995*) It is an aspartic endo-protease that is ubiquitously distributed in lysosomes. Cathepsin D is initially synthesized as a 52kDa precursor protein on rough endoplasmic reticulum, and as such it has no enzymatically activity. By a proteolytic process, it is cleaved into an 46kDa active two-chain form. (*Talieri M, et al., 2004*) The main function of cathepsin D is to degrade protein in lysosomes in an acidic pH. Also, in some specialized cells, it can make the activate proteins by clearing the inactive precursors. ( *Liaudet-Coopman E, et al., 2005*)

Nowadays, the most discussed role of Cathepsin D is its correlation with cancer. Several reports have indicated that expression of this enzyme directly correlates with the prognosis of patients in cancers of various organs. This is because its ability to stimulate cancer cell proliferation. The production of cathepsin D increases the invasive potential of the cancer cells, resulting in the increase of the possibility for metastasis. Also, it can degrade the extra-cellular matrix and activate some other proteases which have also been considered correlated with tumor progression, such as cathepin B and L (*Oh-e H, et al., 2001*). However, Liaudet-Coopman E and colleagues' research results showed cathepsin D may act as extra-cellular binding protein instead of a protease by triggering an unidentified cell surface receptor directly or indirectly. It can, not only stimulate cancer cell proliferation, but also tumor angiogenesis. Furthermore, it is considered that, by interacting with the M6P/IGFII-receptor, whose function is well known in the transport of different ligands via the endosomal pathways, cathepsin D promote tumor cell proliferation. (*Liaudet-Coopman E, et al., 2005*) Some other studies have showed that cathepsin D is a key positive mediator of induced-apoptosis. Therefore cathepsin D may have a dual role in apoptosis. (*Liaudet-Coopman E, et al., 2005*)

This is in agreement with our research data, cathepsin D is over expressed in cancer tissues compared to normal tissues. The average 'Cancer'/'Normal' ratio of %IOD is quite good (1.7) though the protein quantity in both colorectal cancer biopsies and 'normal' groups is relatively low. Some other reports also indicated the cathepsin D activity rather than content is correlated with the malignant progression of colorectal cancer. Compared with the continuous increasing of the content, the activity is increasing from normal mucosa to paired tumour cytocol but then decreasing when the tumour becomes bigger. (*Tumminello FM, et al., 1995*) So, combining the change of amount and the activity of the cathepsin D protein may give us clearer diagnostic evidence.

### Galectin-1

Galectin-1 belongs to galectin family of structurally related proteins distributed widely in all living organisms. This family is defined by having at least one carbohydrate-recognition domain and their affinity for β- galactose- containing ologoaccharides. (*Danguy A, et al., 2002; Rabinovich GA, 2005)*

As a galactose-binding protein, galectin-1 was demonstrated to be an effective mitogen for a number of cell types and many growth-regulatory phenomena have been ascribed to galectin-1. For example, by interacting with a cellular β- galactoside ligand, it may promote a mitogenic or a cytostatic reponse. It can interact with a unknown ligand to inhibit proliferation of many cell types, both normal and tumor cells independently of β- galactoside binding. Also, though some tumour cells are resistant to its effects on the growth and metastasis, the galectin-1 mediated growth inhibition or apoptosis in their surrounding tissues may influence the growth and survival of the tumour cells. (*Scott K, et al., 2004*)

The over- expression of galectin -1 in cancer cells compared with normal cells has been reported in many human organs, such as bladder, thyroid, colon et al. Also, a marked increase in cytoplasmic galectin-1 is reported during the disease progression in human colon cancer. Another important observation is that the increase expression of galectin-1 is associated with an alteration of the intracellular location of its expression. During the development of the tumor, its expression becomes increasingly in cytoplasmic as opposed to the nuclear localization seen in normal colon tissues *(Hittelet A, et al., 2003)* Another study also indicated that galectin-1 over expression in colorectal mucosa is associated with the neoplastic progression (*Sanjuan X, et al., 1997*). Therefore, galectin-1 can be a key role for the malignant behaviour of colon cancer cells. *(Hittelet A, et al., 2003)* Our data shows exactly the same expression tendency with an average 'Cancer'/'Normal' ratio of %IOD 1.61. So galectins-1 can be a fairly good diagnostic marker for colorectal cancer.

Another member in galectin family - galectin-3 is also reported to be involved in malignant progression of colon cancer and may be an even better marker for clinic diagnoses. *(Hittelet A, et al., 2003)*

### Proteasome subunit alpha type 5

The proteasome is a highly conserved, abundant multicatalytic enzyme complex present in the cytoplasm and nucleus of all eukaryotic cells. (*Zavrski I, et al., 2005; Dalton WS, 2004*) Its main function is to eliminate cellular proteins, not only removing damaged and misfolded proteins, but also various proteins with important cellular roles, such as regulation of cell cycle, cell differentiation, apoptosis, stress response, et al,. It is reported that over 80% of all cellular proteins are degraded by the proteasome. (*Dalton WS, 2004*) Because of the critical roles of these substrates in cellular metabolism, the proteasome is thus an essential component in the cell.

Research carried out on nuclear factor-κB (NF- κB) have demonstrated the effects of proteasome inhibition which results in the stability and accumulation of its unwanted substrates. NF- κB is a transcription factor which is retained in the cytoplasm when it is combined with I-κB, an inhibitory partner protein. When I-κB undergoes regulated serine phosphorylation, it is degraded in the proteasome. Once liberated from I-κB, NF- κB moves into the nucleus, and then it controls transcription genes involved in the cell differentiation, growth and apoptosis. Also, it promotes cell proliferation and oncogenesis. Inhibition of I-κB degradation by proteasome inhibitors can keep NF- κB in cytoplasm, thus prevent its interaction with nuclear DNA. Because the inhibitors of I-κB phosphorylation cannot fully prevent cell proliferation, it indicates that the effects of proteasome inhibitors are multi-target pathways. (*Mitchell BS. 2003; Zavrski I, et al., 2005)* Bortezomib, a reversible boronic acid dipeptide proteasome inhibitor, is the first proteasome inhibitor that is being tested cancer clinical trails.

So, though it is novel, proteasome is a fairly good target for various cancer therapies, including colorectal cancer. The studies on colon cancer cell lines (SW48, SW116) and colon cancer specimens both demonstrated that proteasome inhibitors can inhibit cell growth and induce apoptosis. (*Hochwald SN, et al., 2003*)

Up-regulation of proteasome in our study can indicate the development of colorectal cancer in accordance with its known cellular role mentioned before.

### Serum albumin

Human Serum Albumin is a versatile transport protein in the blood. Its primary function is to carry hydrophobic fatty acid molecules around the blood-stream. It also binds a lot of other natural substances and a wide range of drug molecules.

The protein is composed of a single polypeptide chain. It is 67% alpha-helical and contains no beta-sheet structure. It is composed of three homologous domains which assemble to form a heart-shaped molecule. (*He XM, et al., 1992*)

Because of the wide distribution and its possible ability to reflect nutritional status and protein intake (*Knekt P, et al., 2000*), serum albumin is considered to associate with various diseases, including cancers.

One study done by Knekt and colleagues indicated that higher serum albumin levels are associated with a higher risk factor for cancer of the distal colon, but not for cancer of the proximal colon or rectum. In our data, it is also up-regulated in colorectal cancer with an average 'Cancer'/'Normal' ratio of %IOD 1.82. One explanation is the high protein intake or other dietary factors in and around the tumors results in a high concentration of serum albumin. (*Knekt P, et al., 2000*)

Among the nine proteins, seven of them have the same expression tendency as previous reports. No expression comparison between colorectal normal and cancer tissues was reported for the remaining two proteins, L-plastin and Apolipoprotein A-I. In other words, our data agree completely with previous publications relating to markers for colorectal cancer.

In addition to these 9 proteins we have identified 57 other proteins which have never been linked to colorectal cancer and most or all can be proposed to be correlation with colorectal cancer.

### Conclusions drawn based on the above experimental section

In total 298 proteins were selected by 2DGE, and of these 125 were up regulated and therefore potential diagnostic markers. Using 2DGE and MS, 66 proteins with significant expression difference between normal colorectal tissues and colorectal cancer tissues were identified. Nine of these proteins were previously reported as markers, targets or having influence on colorectal cancer.

It demonstrated that 2DGE combined with MS techniques is an excellent approach to find the markers or target proteins of colorectal cancer. Of the 9 proteins which were found in these studies and were also found associated with colorectal cancer, our data agree with and extend all the previous observations. Not only the nine proteins that have been previously reported, but all the proteins identified can also be used for diagnostic purposes. Thus the results obtained have a very high value in a clinical setting.

### References

Adelina Rogowska-Wrzesinka , Larsen PM Blomberg A., Gorg A., Roepstorff P., Norbeck J. Fey SJ., Comparison of the proteomes of three yeast wild type strains: CEN.PK2, FY1679 and W303; Comparative and Functional Genomics 2001;2:207-225 Adenis A, Huet G, Zerimech F, Hecquet B, Balduyck M, Peyrat JP. Cathepsin B, L, and D activities in colorectal carcinomas: relationship with clinico-pathological parameters. Cancer Lett. 1995 Sep 25;96(2):267-75.

Aebersold R, Mann M Mass spectrometry-based proteomics Nature 2003 Mar13, 422(6928):198-207

Arpin M, Friederich E, Algrain M, Vernel F, Louvard D. Functional differences between L- and T-plastin isoforms. J Cell Biol. 1994 Dec;127(6 Pt 2):1995-2008.

Boel Lanne, Oleg Panfilov Protein staining influences the quality of mass spectra obtained by peptide mass fingerprinting after separation on 2-D gels. A comparison of staining with Coomassie Brilliant Blue and Sypro Ruby. Journal of Proteome research 2005, 4,175-179

Bradford MM. A rapid and sensitive method for the quantitation of microgram quantities of protein utilizing the principle of protein-dye binding. Anal Biochem. 1976 May 7;72:248-54.

Chabowski A, Skrzydlewska E, Sulkowska M, Famulski W, Zalewski B, Gnojnicki I, Kisielewski W. Cathepsin D activity in colorectal cancer. Rocz Akad Med Bialymst. 2001;46:38-46.

Coutinho AK, Rocha Lima CM. Metastatic colorectal cancer: systemic treatment in the new millennium. Cancer Control. 2003 May-Jun;10(3):224-38.

Dalton WS. The proteasome. Semin Oncol. 2004 Dec;31 (6 Suppl 16):3-9; discussion 33. Review.

Danguy A, Camby I, Kiss R. Galectins and cancer. Biochim Biophys Acta. 2002 Sep 19;1572(2-3):285-93. Review.

Etzioni R, Urban N, Ramsey S, McIntosh M, Schwartz S, Reid B, Radich J, Anderson G, Hartwell L. The case for early detection. Nat Rev Cancer. 2003 Apr;3(4):243-52.

Fey SJ, Nawrocki A, Larsen MR, Gorg A, Roepstorff P, Skews GN, Williams R, Larsen PM. Proteome analysis of Saccharomyces cerevisiae: a methodological outline. Electrophoresis. 1997 Aug;18(8):1361-72.

Fey SJ, Mose Larsen P. 2D or not 2D Curr Opin Chem Biol. 2001 Feb; 5(1):26-33.

Frank PG, Marcel YL.Apolipoprotein A-I: structure-function relationships. J Lipid Res. 2000 Jun; 41 (6):853-72. Review

Gartner W, Lang W, Leutmetzer F, Domanovits H, Waldhausl W, Wagner L. Cerebral expression and serum detectability of secretagogin, a recently cloned EF-hand Ca(2+)-binding protein. Cereb Cortex. 2001 Dec;11(12):1161-9.

Haraguchi M, Komuta K, Akashi A, Furui J, Kanematsu T. Occurrence of hematogenous metastasis and serum levels of thymidine phosphorylase in colorectal cancer. Oncol Rep. 2003 Sep-Oct; 10(5):1207-12.

He XM, Carter DC. Atomic structure and chemistry of human serum albumin. Nature. 1992 Jul 16;358(6383):209-15. Erratum in: Nature 1993 Jul 22;364(6435):362.

Hittelet A, Legendre H, Nagy N, Bronckart Y, Pector JC, Salmon I, Yeaton P, Gabius HJ, Kiss R, Camby I. Upregulation of galectins-1 and -3 in human colon cancer and their role in regulating cell migration. Int J Cancer. 2003 Jan 20;103(3):370-9.

Hochwald SN, Lind DS, Malaty J, Copeland EM 3rd, Moldawer LL, MacKay SL. Antineoplastic therapy in colorectal cancer through proteasome inhibition. Am Surg. 2003 Jan;69(1):15-23.

Hoffman E.D. Stroobant V. Mass spectrometry, Principle and Applications JohnWiley & Sons, Ltd, 2001, Second edition

Hyka N, Dayer JM, Modoux C, Kohno T, Edwards CK 3rd, Roux-Lombard P, Burger D. Apolipoprotein A-I inhibits the production of interleukin-1beta and tumor necrosis factor-alpha by blocking contact-mediated activation of monocytes by T lymphocytes. Blood. 2001 Apr 15;97(8):2381-9.

Jensen ON, Shevchenko, A., Mann,M. Protein analysis by mass spectrometry. In protein structure -A practical approach 1997 (2nd edition, T.E. Creighton, ed.) Oxford: IRL Press, Oxford University Press.

Jensen ON, Larsen MR, Roepstorff P. Mass spectrometric identification and microcharacterization of proteins from electrophoretic gels: strategies and applications. Proteins 1998; Suppl 2:74-89

Jemal A, Thomas A, Murray T, Thun M Cancer Statistics CA Cnacer J Clin 2002. 2002 52:23- 47

Kiera N. Berggren et al. An improved formulation of Sypro Ruby protein gel stain: Comparison with the orginal formulation and with a ruthenium II tris (bathophenanthroline disulfonate) formulation Proteomics 2002, 2,486-498

Klose, J. Protein mapping by combined isoelectric focusing and electrophoresis of mouse tissues: A novel approach to testing for induced point mutation in mammals. Humangenetik 1975 26, 231-243

Knekt P, Hakulinen T, Leino A, Heliovaara M, Reunanen A, Stevens R. Serum albumin and colorectal cancer risk. Eur J Clin Nutr. 2000 Jun;54(6):460-2.

Ian R. White et al. A statistical comparison of silver and SYPRO Ruby staining for proteomic analysis Electrophoresis 2004, 25, 3048-3054

Lawson D, Harrison M, Shapland C. Fibroblast transgelin and smooth muscle SM22alpha are the same protein, the expression of which is down-regulated in many cell lines. Cell Motil Cytoskeleton. 1997;38(3):250-7.

Lawrie LC, Dundas SR, Curran S, Murray GI. Liver fatty acid binding protein expression in colorectal neoplasia. Br J Cancer. 2004 May 17;90(10):1955-60.

Liaudet-Coopman E, Beaujouin M, Derocq D, Garcia M, Glondu-Lassis M, Laurent-Matha V, Prebois C, Rochefort H, Vignon F. Cathepsin D: newly discovered functions of a long-standing aspartic protease in cancer and apoptosis. Cancer Lett. 2005 Jul 18;

Lin CS, Chen ZP, Park T, Ghosh K, Leavitt J Characterization of the human L-plastin gene promoter in normal and neoplastic cells. J Biol Chem. 1993 Feb 5; 268(4):2793-801.

Lin CS, Park T, Chen ZP, Leavitt J. Human plastin genes. Comparative gene structure, chromosome location, and differential expression in normal and neoplastic cells. J Biol Chem. 1993 Feb 5; 268(4):2781-92.

L.M. Franks, N.M. Teich Introduction to the cellular and molecular biology of cancer Third Edition 1997 Oxford University Press

Lynth HT, de la Chapelle A Hereditary colorectal cancer N.Engl.J.Med. 2003. 348(10):919-32

Mann M, Talbo G. Developments in matrix-assisted laser desorption /ionization peptide mass spectrometry Curr. Opin Biotechnol 1996, 7 11

Marcel YL, Kiss RS.Structure-function relationships of apolipoprotein A-I: a flexible protein with dynamic lipid associations. Curr Opin Lipidol. 2003 Apr;14(2):151-7. Review.

McKerrow JH, Bhargava V, Hansell E, Huling S, Kuwahara T, Matley M, Coussens L, Warren R. A functional proteomics screen of proteases in colorectal carcinoma. Mol Med. 2000 May;6(5):450-60.

Miettinen M. Keratin 20: immunohistochemical marker for gastrointestinal, urothelial, and Merkel cell carcinomas. Mod Pathol. 1995 May;8(4):384-8.

Mitchell BS. The proteasome-an emerging therapeutic target in cancer. N Engl J Med. 2003 Jun 26;348(26):2597-8.

Moll R, Lowe A, Laufer J, Franke WW. Cytokeratin 20 in human carcinomas. A new histodiagnostic marker detected by monoclonal antibodies. Am J Pathol. 1992 Feb;140(2):427-47.

Moll R, Zimbelmann R, Goldschmidt MD, Keith M, Laufer J, Kasper M, Koch PJ, Franke WW. The human gene encoding cytokeratin 20 and its expression during fetal development and in gastrointestinal carcinomas. Differentiation. 1993 Jun;53(2):75-93. Nawrocki A, Fey SJ, Goffeau A, Roepstorff P, Larsen PM. The effects of transcription regulating genes PDR1, pdr1-3 and PDR3 in pleiotropic drug resistance. Proteomics. 2001 Aug;1(8):1022-32. Erratum in: Proteomics 2001 Oct;1(10):1340-1.

Normanno N, De Luca A, Castaldo A, Casamassimi A, Di Popolo A, Zarrilli R, Porcellini A, Acquaviva AM, Avvedimento VE, Pignata S. Apolipoprotein A-I reverse transcriptase-polymerase chain reaction analysis for detection of hematogenous colon cancer dissemination. Int J Oncol. 1998 Sep; 13(3):443-7.

O'Farrell, P.H. High resolution two-dimensional electrophoresis of proteins. J. Biol. Chem. 1975 250, 4007-4021

Oh-e H, Tanaka S, Kitadai Y, Shimamoto F, Yoshihara M, Haruma K. Cathepsin D expression as a possible predictor of lymph node metastasis in submucosal colorectal cancer. Eur J Cancer. 2001 Jan;37(2):180-8.

Otsuka M, Kato M, Yoshikawa T, Chen H, Brown EJ, Masuho Y, Omata M, Seki N. Differential expression of the L-plastin gene in human colorectal cancer progression and metastasis. Biochem Biophys Res Commun. 2001 Dec 14;289(4):876-81.

Rabilloud T. et al. A comparison between Sypro Ruby and ruthenium II tris (bathophenanthroline disulfonate) as fluorescent stains for protein detection in gels Proteomics 2001, 1,699-704

Rabinovich GA. Galectin-1 as a potential cancer target. Br J Cancer. 2005 Apr 11;92(7):1188-92. Review.

Raffel J, Bhattacharyya AK, Gallegos A, Cui H, Einspahr JG, Alberts DS, Powis G. Increased expression of thioredoxin-1 in human colorectal cancer is associated with decreased patient survival. J Lab Clin Med. 2003 Jul;142(1):46-51.

Reiner Westermeier Electrophoresis in Practice Second Edition 1997

Robert K. Scopes Protein Purification Principle and practice Third Edition 1993

Sanjuan X, Fernandez PL, Castells A, Castronovo V, van den Brule F, Liu FT, Cardesa A, Campo E. Differential expression of galectin 3 and galectin 1 in colorectal cancer progression. Gastroenterology. 1997 Dec;113(6):1906-15.

Schwerer MJ, Baczako K. Immunohistochemical evaluation of keratin 20 expression in intestinal metaplasia types I to III. J Clin Pathol. 1996 Oct;49(10):791-4.

Scott K, Weinberg C. Galectin-1: a bifunctional regulator of cellular proliferation. Glycoconj J. 2004;19(7-9):467-77. Review.

Segrest JP, Li L, Anantharamaiah GM, Harvey SC, Liadaki KN, Zannis V.Structure and function of apolipoprotein A-I and high-density lipoprotein. Curr Opin Lipidol. 2000 Apr;11(2):105-15. Review.

Shapland C, Hsuan JJ, Totty NF, Lawson D. Purification and properties of transgelin: a transformation and shape change sensitive actin-gelling protein. J Cell Biol. 1993 Jun;121(5):1065-73.

Shields JM, Rogers-Graham K, Der CJ. Loss of transgelin in breast and colon tumors and in RIE-1 cells by Ras deregulation of gene expression through Raf-independent pathways. J Biol Chem. 2002 Mar 22;277(12):9790-9. Epub 2001 Dec 28.

Shiwa M, Nishimura Y, Wakatabe R, Fukawa A, Arikuni H, Ota H, Kato Y, Yamori T. Rapid discovery and identification of a tissue-specific tumor biomarker from 39 human cancer cell lines using the SELDI ProteinChip platform. Biochem Biophys Res Commun. 2003 Sep 12;309(1):18-25.

Storch J, Thumser AE. The fatty acid transport function of fatty acid-binding proteins. Biochim Biophys Acta. 2000 Jun 26;1486(1):28-44. Review.

Sun D, Zhao YY, Dai SP, Fang K, Dong LY, Ding Q. Cloning and analysis of human alpha-1B glycoprotein precursor gene: a novel member of human immunoglobulin superfamily Yi Chuan Xue Bao. 2002 Apr;29(4):299-302. Japanese.

Talieri M, Papadopoulou S, Scorilas A, Xynopoulos D, Arnogianaki N, Plataniotis G, Yotis J, Agnanti N.Cathepsin B and cathepsin D expression in the progression of colorectal adenoma to carcinoma. Cancer Lett. 2004 Mar 8;205(1):97-106.

Tom Berkelman, Tirra Stestedt 2-D Electrophoresis using immobilized pH gradientsPrinciples& Methods 1998

Tumminello FM, Gebbia N, Pizzolanti G, Russo A, Bazan V, Leto G.Cathepsin D content in colorectal cancer. Correlation with cathepsin D activity and other biological parameters: a preliminary report. Oncology. 1995 May-Jun;52(3):237-42.

van den Brule F, Califice S, Castronovo V. Expression of galectins in cancer: a critical review. Glycoconj J. 2004;19(7-9):537-42. Review.

Wang Y, Morrow JS. Identification and characterization of human SLP-2, a novel homologue of stomatin (band 7.2b) present in erythrocytes and other tissues. J Biol Chem. 2000 Mar 17;275(11):8062-71.

Wildi S, Kleeff J, Maruyama H, Maurer CA, Friess H, Buchler MW, Lander AD, Korc M. Characterization of cytokeratin 20 expression in pancreatic and colorectal cancer. Clin Cancer Res. 1999 Oct;5(10):2840-7.

Yamazaki T, Kanda T, Sakai Y, Hatakeyama K. Liver fatty acid-binding protein is a new prognostic factor for hepatic resection of colorectal cancer metastases. J Surg Oncol. 1999 Oct;72(2):83-7.

Yergey A.L., Coorssen J.R.,Backlund P.S. Jr. Et al., De novo sequencing of peptides using MALDI/TOF-TOF J Am Soc Mass Spectrom 2002 Jul, 13(7): 784-91

Yun K, Merrie AE, Gunn J, Phillips LV, McCall JL. Keratin 20 is a specific marker of submicroscopic lymph node metastases in colorectal cancer: validation by K-RAS mutations. J Pathol. 2000 May;191(1):21-6.

Zavrski I, Jakob C, Schmid P, Krebbel H, Kaiser M, Fleissner C, Rosche M, Possinger K, Sezer O. Proteasome: an emerging target for cancer therapy. Anticancer Drugs. 2005 Jun;16(5):475-81. Review.

Zheng J, Rudra-Ganguly N, Miller GJ, Moffatt KA, Cote RJ, Roy-Burman P. Steroid hormone induction and expression patterns of L-plastin in normal and carcinomatous prostate tissues. Am J Pathol. 1997 Jun;150(6):2009-18.

Zhou Q, Toivola DM, Feng N, Greenberg HB, Franke WW, Omary MB. Keratin 20 helps maintain intermediate filament organization in intestinal epithelia. Mol Biol Cell. 2003 Jul;14(7):2959-71. Epub 2003 Apr 4. http://www.lerner.ccf.org/services/storm/ http://www.humpath.com/article.php3?id article=205 http://www.medinnovation.de/background/hsa.htm

## Claims

1. A method for diagnosing colorectal cancer in a human, the method comprising establishing the increased expression of HSP 90-beta (P08238) in a biological sample from said human.

2. A method according to claim 1, wherein the biological sample is selected from the group consisting of urine, feces, blood, saliva, lymphatic fluids, and tissue.

3. A method according to claim 2, wherein the tissue is taken from the colon and/or rectum.

4. A test kit for diagnosing colorectal cancer or a genetic predisposition for colorectal cancer in a human, comprising:
a) a binding means which specifically binds to HSP 90-beta (P08238);
b) a means for detecting binding, if any, or the level of binding, of the binding means to HSP 90-beta (P08238);
c) a means for correlating whether binding, if any, or the level of binding, to said binding means is indicative of the individual mammal having a significantly higher likelyhood of having colorectal cancer or a genetic predisposition for having colorectal cancer.

5. A method for determining the effect of a substance, the method comprising using a human, which has been established to be an individual having a high likelihood of having colorectal cancer or a genetic predisposition for having colorectal cancer by use of a method according to any one of claims 1 to 3, the method comprising administering the substance to the individual and determining the effect of the substance.
